# EUROPEAN PATENT APPLICATION

(11) **EP 4 721 746 A1**
(43) Date of publication of application: **08.04.2026**
(21) Application number: 24815178.9
(22) Date of filing: 13.05.2024
(51) Int. Cl.: A61K 31/675, A61P 35/00, A61P 35/02, C12Q 1/6851, G01N 33/53, G01N 33/574

(54) **TREATMENT OF MALIGNANT TUMOR**

(30) Priority: 31.05.2023 JP 2023090376; 04.03.2024 JP 2024032469
(71) Applicant: SymBio Pharmaceuticals Limited, Tokyo 105-0001 (JP)
(72) Inventor: HAZAMA, Masatoshi, Tokyo 105-0001 (JP); FUKUSHIMA, Koji, Tokyo 105-0001 (JP); CHAN, Jason, Singapore 822312 (SG); ONG, Choon Kiat, Singapore 510146 (SG)
(74) Representative: Gulde & Partner
(86) International application number: PCT/JP2024/017637
(87) International publication number: WO 2024/247694

(57) **Abstract**

Provided is a method related to the treatment of a malignant tumor. A pharmaceutical composition for the treatment of a malignant tumor is used, the composition comprising brincidofovir, a pharmaceutically acceptable salt thereof, or a solvate thereof, wherein a subject of the treatment has a malignant tumor with low TLE1 expression. A pharmaceutical composition for the treatment of a malignant tumor may also be used, the composition comprising brincidofovir, a pharmaceutically acceptable salt thereof, or a solvate thereof, wherein a subject of the treatment is a subject identified based on an expression level of TLE1 as an indicator. A method may also be used for predicting sensitivity to brincidofovir, a pharmaceutically acceptable salt thereof, or a solvate thereof in a subject with a malignant tumor, the method comprising testing an expression level of TLE1 in a sample derived from the subject with the malignant tumor.

## Description

### Technical Field

The technical field of the present invention relates to the treatment of malignant tumors.

### Background Art

Brincidofovir (BCV) has been approved by the U.S. Food and Drug Administration (FDA) in 2021 as a drug for treating smallpox. Non-Patent Literature 1 describes the benefit-risk assessment of BCV for the treatment of smallpox. In addition, Non-Patent Literature 2 states that monkeypox began to spread in May 2022 and that the use of BCV is recommended for the management of confirmed cases.

### Citation List

### Non-Patent Literature

Non-Patent Literature 1: "Benefit-risk assessment for brincidofovir for the treatment of smallpox: U.S. Food and Drug Administration's Evaluation" Chan-Tack et al., Antiviral Res. 2021 Nov;195:105182.
Non-Patent Literature 2: "Monkeypox: A Mini-Review on the Globally Emerging Orthopoxvirus" Evans et al., Int J Environ Res Public Health. 2022 Nov 25;19(23):15684.

### Summary of Invention

### Technical Problem

Many unknown points remained regarding the therapeutic effects and treatment methods for malignant tumors using BCV.

On the other hand, as described in the Examples below, the present inventors have found that BCV sensitivity in malignant tumors is associated with the expression level of TLE1. It was shown that implementing BCV treatment by utilizing this finding makes it possible to improve the therapeutic effect or increase the probability of a therapeutic effect appearing.

### Solution to Problem

An aspect of the present invention provides a pharmaceutical composition for treatment of a malignant tumor, comprising brincidofovir, a pharmaceutically acceptable salt thereof, or a solvate thereof, wherein a subject of the treatment has a malignant tumor with low TLE1 expression. This pharmaceutical composition can be used to perform an appropriate treatment with brincidofovir, a pharmaceutically acceptable salt thereof, or a solvate thereof.

An aspect of the present invention provides a pharmaceutical composition for treatment of a malignant tumor, comprising brincidofovir, a pharmaceutically acceptable salt thereof, or a solvate thereof, wherein a subject of the treatment is a subject identified based on an expression level of TLE1 as an indicator. This pharmaceutical composition can be used to perform an appropriate treatment with brincidofovir, a pharmaceutically acceptable salt thereof, or a solvate thereof.

An aspect of the present invention provides a composition for use in predicting sensitivity to brincidofovir, a pharmaceutically acceptable salt thereof, or a solvate thereof in a subject with a malignant tumor, the composition comprising a test reagent for an expression level of TLE1. This composition can be used to predict sensitivity.

An aspect of the present invention provides a composition for use in determining a subject with a malignant tumor to be administered brincidofovir, a pharmaceutically acceptable salt thereof, or a solvate thereof, the composition comprising a test reagent for an expression level of TLE1. This composition can be used to determine a subject suitable for administration of brincidofovir, a pharmaceutically acceptable salt thereof, or a solvate thereof.

An aspect of the present invention provides a composition for use in diagnosing whether a subject is a subject having a malignant tumor with high sensitivity to brincidofovir, a pharmaceutically acceptable salt thereof, or a solvate thereof, the composition comprising a test reagent for an expression level of TLE1. This composition can be used to diagnose whether a subject is a subject having a malignant tumor with high sensitivity.

An aspect of the present invention provides a method for predicting sensitivity to brincidofovir, a pharmaceutically acceptable salt thereof, or a solvate thereof in a subject with a malignant tumor, the method comprising testing an expression level of TLE1 in a sample derived from the subject with the malignant tumor. This method can be used to predict sensitivity.

An aspect of the present invention provides a method for determining a subject with a malignant tumor to be administered brincidofovir, a pharmaceutically acceptable salt thereof, or a solvate thereof, the method comprising testing an expression level of TLE1 in a sample derived from the subject with the malignant tumor. This method can be used to determine a subject suitable for administration of brincidofovir, a pharmaceutically acceptable salt thereof, or a solvate thereof.

An aspect of the present invention provides a method for diagnosing whether a subject is a subject having a malignant tumor with high sensitivity to brincidofovir, a pharmaceutically acceptable salt thereof, or a solvate thereof, the method comprising testing an expression level of TLE1 in a sample derived from the subject with the malignant tumor. This method can be used to diagnose whether a subject is a subject having a malignant tumor with high sensitivity.

An aspect of the present invention provides a composition for use in predicting a prognosis of a malignant tumor, the composition comprising a test reagent for an expression level of TLE1. This composition can be used to predict a prognosis.

An aspect of the present invention provides a method for predicting a prognosis of an NK/T lymphoma or a B-cell lymphoma using an expression level of TLE1 in a sample from a subject with a malignant tumor as an indicator. This method can be used to predict a prognosis.

An aspect of the present invention provides a composition for use in selecting a treatment method for a malignant tumor, the composition comprising a test reagent for an expression level of TLE1. This composition can be used to select an appropriate treatment method.

An aspect of the present invention provides a method for selecting a treatment method for a malignant tumor using an expression level of TLE1 in a sample from a subject as an indicator. This method can be used to select an appropriate treatment method.

An aspect of the present invention provides a pharmaceutical composition for treatment of a malignant tumor, comprising brincidofovir, a pharmaceutically acceptable salt thereof, or a solvate thereof, wherein the malignant tumor is a T-cell lymphoma. This pharmaceutical composition can be used to perform an appropriate treatment with brincidofovir, a pharmaceutically acceptable salt thereof, or a solvate thereof.

### Brief Description of Drawings

[Figure 1] Fig. 1 shows the results of an examination of the effect of BCV on the viability of NK/T-cell lymphoma cell lines.
[Figure 2A-D] Figure 2A-D show the results of administration of BCV to NSG mice implanted with NK/T-cell lymphoma cells (NK-S1). Fig. 2A shows the results of an examination of tumor sizes on Days 1, 5, 8, 12, and 15. Fig. 2B shows the results of an examination of body weights on Days 1, 5, 8, 12, and 15. Fig. 2C shows the measurement results of tumor weights. In the bar graph, the bars, from left to right, represent vehicle administration and BCV administration, respectively. Fig. 2D shows a photograph of tumors collected from the respective individuals.

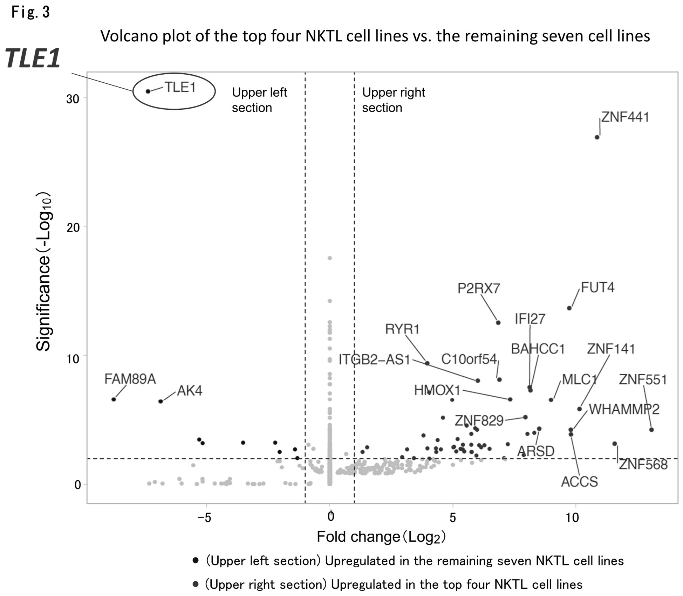

[Figure 3] Fig. 3 shows the results of a whole transcriptome sequence analysis of 11 NK/T-cell lymphoma cell lines. The plot of Fig. 3 shows the relative relationship in a comparison between the top four cell lines and the remaining seven cell lines. Fig. 3 is divided into six sections by dotted lines. The circles in the upper left section containing TLE1, FAM89A, AK4, etc. indicate genes with increased expression in the remaining seven cell lines (i.e., genes with decreased expression in the top four cell lines), and the circles in the upper right section containing ZNF441, ZNF141, etc. indicate genes with increased expression in the top four cell lines. The other four sections contain genes with an expression difference of two-fold or less, or relatively weak genes with a significance of the association of 0.01 or more, between the top four cell lines and the remaining seven cell lines. In the present description and drawings, NKTL has the same meaning as NK/T-cell lymphoma.
[Figure 4] Fig. 4 shows the results of an examination of the protein expression level of TLE1.
[Figure 5] Fig. 5 shows the results of an analysis of gene mutations.
[Figure 6A-B] Figs. 6A-B show the results of a gene set expression analysis. Fig. 6A shows the gene sets that exhibited increased expression in the top four cell lines. Fig. 6B shows the enrichment plot of MYC TARGETS V2.
[Figure 7A-B] Figs. 7A-B show the results of an examination of the effect of the TLE1 expression level on prognosis. Fig. 7A shows the results of an examination of progression-free survival. Fig. 7B shows the results of an examination of overall survival.
[Figure 8] Fig. 8 shows the results of a gene set expression analysis of an NK/T-cell lymphoma patient cohort.
[Figure 9] Fig. 9 shows the results of an examination of the effect of BCV on the viability of T-cell lymphoma cell lines.
[Figure 10A-D] Figs. 10A-D show the results of administration of BCV to NSG mice implanted with T-cell lymphoma cells (Z4739). Fig. 10A shows the results of an examination of tumor sizes on Days 4, 7, 11, 18, and 21. Fig. 10B shows the results of an examination of body weights on Days 1, 4, 7, 11, 18, and 21. Fig. 10C shows the measurement results of tumor weights. In the bar graph, the bars, from left to right, represent vehicle administration and BCV administration, respectively. Fig. 10D shows a photograph of tumors collected from the respective individuals.
[Figure 11] Fig. 11 shows the results of a whole transcriptome sequence analysis of (i) the top four most BCV-sensitive cell lines described in Example 1, (ii) the remaining seven cell lines, and (iii) the 11 cell lines that showed high sensitivity to BCV as described in Example 9.
[Figure 12A-C] Figs. 12A-C show the results of an examination of the effect of BCV on gene expression in T-cell lymphoma cell lines. Fig. 12A shows the results of an examination of the normalized enrichment score. Fig. 12B shows the enrichment plots of MYC TARGETS V2 and V1. Fig. 12C shows the results of an examination of the gene expression profile.
[Figure 13] Fig. 13 shows the results of an examination of the effect of BCV on gene expression in T-cell lymphoma cell lines (results of Western Blotting).
[Figure 14] Fig. 14 shows the results of an examination of the effect of BCV on the viability of B-cell lymphoma cell lines.
[Figure 15A-D] Figs. 15A-D show the results of administration of BCV to NSG mice implanted with B-cell lymphoma cells (OCI-LY19). Fig. 15A shows the results of an examination of tumor sizes on Days 1, 5, 6, and 7. Fig. 15B shows the results of an examination of body weights on Days 1, 5, 6, and 7. Fig. 15C shows the measurement results of tumor weights. In the bar graph, the bars, from left to right, represent vehicle administration and BCV administration, respectively. Fig. 15D shows a photograph of tumors collected from the respective individuals.

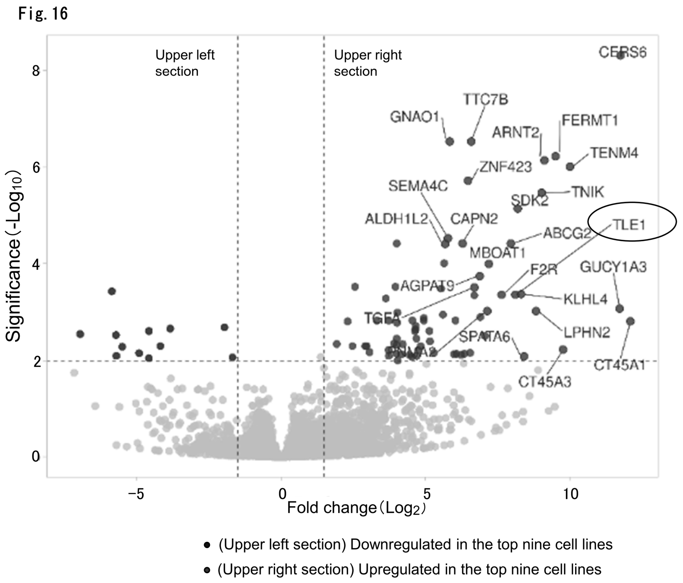

[Figure 16] Fig. 16 shows the results of a whole transcriptome sequence analysis of 19 B-cell lymphoma cell lines. The plot of Fig. 16 shows the relative relationship in a comparison between the top nine cell lines and the remaining 10 cell lines. Fig. 16 is divided into six sections by dotted lines. The circles in the upper left section indicate genes with decreased expression in the top nine cell lines, and the circles in the upper right section containing TLE1, CERS6, etc. indicate genes with increased expression in the top nine cell lines. The other four sections contain genes with an expression difference of two-fold or less, or relatively weak genes with a significance of the association of 0.01 or more, between the top nine cell lines and the remaining 10 cell lines.
[Figure 17A-B] Figs. 17A-B show the results of an examination of the effect of the TLE1 expression level on prognosis. Fig. 17A shows the results of an examination of overall survival based on GSE11318 (Lenz et al., Proc Natl Acad Sci U S A. 2008 Sep 9;105(36):13520-5.). Fig. 17B shows the results of an examination of overall survival based on GSE10846 (Lenz et al., N Engl J Med. 2008 Nov 27;359(22):2313-23.).

### Description of Embodiments

Hereinafter, embodiments of the present invention will be described in detail. Note that repeated descriptions of similar content are omitted where appropriate in order to avoid redundancy.

### (1) Method

An embodiment of the present invention provides a method for treating a malignant tumor, comprising administering brincidofovir (BCV), a pharmaceutically acceptable salt thereof, or a solvate thereof to a subject having a malignant tumor. The subject may be a subject having a malignant tumor with low TLE1 expression, or a subject identified based on an expression level of TLE1 as an indicator. In the Examples described below, BCV sensitivity in malignant tumors was associated with the expression level of TLE1. For example, it was shown that NK/T-cell lymphomas or T-cell lymphomas with a low expression level of TLE1 have high sensitivity to BCV. By utilizing this method, it is possible to administer BCV to a subject who is expected to have low TLE1 expression and high sensitivity to BCV, and in that case, an improvement of the therapeutic effect or an increase in the probability of a therapeutic effect appearing can be expected, as compared to when BCV is administered to an unspecified subject. In addition, by utilizing this method, sensitivity can be predicted using the expression level of TLE1 as an indicator, and a more appropriate BCV treatment can be performed. For example, if a subject's TLE1 expression level is low and the subject is predicted to have high sensitivity, treatment may be performed by administering BCV, or by administering BCV at a low dose. Also, for example, if a subject's TLE1 expression level is high and the subject is predicted to have low sensitivity, treatment may be performed by not administering BCV, or by administering BCV at a high dose. In addition, as described in the Examples below, the prognosis was particularly poor in patients with BCV-untreated NK/T-cell lymphoma with low TLE1 expression. Therefore, BCV can be used for the treatment of malignant tumors with a poor prognosis. In addition, in the Examples described below, it was shown that B-cell lymphomas with a high expression level of TLE1 have high sensitivity to BCV. Therefore, by utilizing this method, it is possible to administer BCV to a subject who is expected to have high TLE1 expression and high sensitivity to BCV, and in that case, an improvement of the therapeutic effect or an increase in the probability of a therapeutic effect appearing can be expected, as compared to when BCV is administered to an unspecified subject. In addition, by utilizing this method, for malignant tumors with high TLE1 expression and high sensitivity to BCV, sensitivity can be predicted using the expression level of TLE1 as an indicator, and a more appropriate BCV treatment can be performed. For example, if a subject's TLE1 expression level is high and the subject is predicted to have high sensitivity, treatment may be performed by administering BCV, or by administering BCV at a low dose. Also, for example, if a subject's TLE1 expression level is low and the subject is predicted to have low sensitivity, treatment may be performed by not administering BCV, or by administering BCV at a high dose. In addition, as described in the Examples below, the prognosis was particularly poor in patients with BCV-untreated B-cell lymphoma with high TLE1 expression. Therefore, BCV can be used for the treatment of malignant tumors with a poor prognosis.

### (2) Method

An embodiment of the present invention provides a method for treating a malignant tumor with a poor prognosis, comprising administering BCV, a pharmaceutically acceptable salt thereof, or a solvate thereof to a subject having a malignant tumor, wherein the subject is a subject identified based on an expression level of TLE1 as an indicator. This method can be used for the treatment of malignant tumors with a poor prognosis.

### (3) Method

An embodiment of the present invention provides a method for treating a malignant tumor, comprising administering BCV, a pharmaceutically acceptable salt thereof, or a solvate thereof to a subject with a malignant tumor (including a subject having a malignant tumor) with low TLE1 expression. By utilizing this method, a particularly high therapeutic effect by BCV is obtained by administering it to a subject having a malignant tumor with low TLE1 expression and high sensitivity to BCV. By utilizing this method, the probability of a therapeutic effect appearing is increased by making a subject who is expected to have high sensitivity to BCV a subject to be treated. An embodiment of the present invention provides a method for treating a malignant tumor, comprising administering BCV, a pharmaceutically acceptable salt thereof, or a solvate thereof to a subject with a malignant tumor with high TLE1 expression. By utilizing this method, a particularly high therapeutic effect by BCV is obtained by administering it to a subject having a malignant tumor with high TLE1 expression and high sensitivity to BCV. By utilizing this method, the probability of a therapeutic effect appearing is increased by making a subject who is expected to have high sensitivity to BCV a subject to be treated.

### (4) Method

An embodiment of the present invention provides a method for inhibiting the proliferation of malignant tumor cells, comprising contacting BCV, a pharmaceutically acceptable salt thereof, or a solvate thereof with malignant tumor cells with a low expression level of TLE1 (malignant tumor cells with low TLE1 expression). By utilizing this method, a particularly high inhibitory effect on the proliferation of malignant tumor cells is obtained by contacting BCV with malignant tumor cells with low TLE1 expression and high sensitivity to BCV. An embodiment of the present invention provides a method for inhibiting the proliferation of malignant tumor cells, comprising contacting BCV, a pharmaceutically acceptable salt thereof, or a solvate thereof with malignant tumor cells with a high expression level of TLE1 (malignant tumor cells with high TLE1 expression). By utilizing this method, a particularly high inhibitory effect on the proliferation of malignant tumor cells is obtained by contacting BCV with malignant tumor cells with high TLE1 expression and high sensitivity to BCV.

### (5) Method

An embodiment of the present invention provides a method for predicting sensitivity to BCV, a pharmaceutically acceptable salt thereof, or a solvate thereof in a subject with a malignant tumor, comprising testing an expression level of TLE1 in a sample derived from the subject with the malignant tumor. This method can be used to predict or identify a subject with high sensitivity or low sensitivity to BCV using the expression level of TLE1 as an indicator. Therefore, by utilizing this method, a more appropriate BCV treatment can be performed. For example, if a subject's TLE1 expression level is low and the subject is predicted to have high sensitivity, treatment may be performed by administering BCV, or by administering BCV at a low dose. For example, if a subject's TLE1 expression level is high and the subject is predicted to have low sensitivity, treatment may be performed by not administering BCV, or by administering BCV at a high dose. For example, if a subject's TLE1 expression level is high and the subject is predicted to have high sensitivity, treatment may be performed by administering BCV, or by administering BCV at a low dose. For example, if a subject's TLE1 expression level is low and the subject is predicted to have low sensitivity, treatment may be performed by not administering BCV, or by administering BCV at a high dose. From another aspect, an embodiment of the present invention provides a method for predicting sensitivity to BCV, a pharmaceutically acceptable salt thereof, or a solvate thereof in a subject with a malignant tumor, comprising testing an expression level of TLE1 in malignant tumor cells derived from the subject with the malignant tumor.

### (6) Method

An embodiment of the present invention provides a method for determining a subject with a malignant tumor to be administered BCV, a pharmaceutically acceptable salt thereof, or a solvate thereof, comprising testing an expression level of TLE1 in a sample derived from the subject with the malignant tumor. By utilizing this method, a more appropriate BCV treatment can be performed. For example, if a subject's TLE1 expression level is low and the subject is predicted to have high sensitivity, the subject may be determined as a subject for administration of BCV, or as a subject for administration at a low dose. For example, if a subject's TLE1 expression level is high and the subject is predicted to have low sensitivity, the subject may be determined as a subject for administration of BCV at a high dose. For example, if a subject's TLE1 expression level is high and the subject is predicted to have high sensitivity, the subject may be determined as a subject for administration of BCV, or as a subject for administration at a low dose. For example, if a subject's TLE1 expression level is low and the subject is predicted to have low sensitivity, the subject may be determined as a subject for administration of BCV at a high dose. The subject to be administered may be a subject who should be administered. From another aspect, an embodiment of the present invention provides a method for selecting a subject with a malignant tumor who is suitable for treatment with BCV, a pharmaceutically acceptable salt thereof, or a solvate thereof, comprising testing an expression level of TLE1 in a sample derived from the subject with the malignant tumor.

### (7) Method

An embodiment of the present invention provides a method for diagnosing whether a subject is a subject having a malignant tumor with high sensitivity to BCV, a pharmaceutically acceptable salt thereof, or a solvate thereof, comprising testing an expression level of TLE1 in a sample derived from the subject with the malignant tumor. This method can be used to diagnose whether a subject is a subject having a malignant tumor with high sensitivity to BCV using the expression level of TLE1 as an indicator. Therefore, by utilizing this method, a more appropriate BCV treatment can be performed. For example, if a subject is diagnosed as being a subject having a malignant tumor with a low expression level of TLE1 and high sensitivity, treatment may be performed by administering BCV, or by administering BCV at a low dose. For example, if a subject is diagnosed as not being a subject having a malignant tumor with a high expression level of TLE1 and high sensitivity, treatment may be performed by not administering BCV, or by administering BCV at a high dose. For example, if a subject is diagnosed as being a subject having a malignant tumor with a high expression level of TLE1 and high sensitivity, treatment may be performed by administering BCV, or by administering BCV at a low dose. For example, if a subject is diagnosed as not being a subject having a malignant tumor with a low expression level of TLE1 and high sensitivity, treatment may be performed by not administering BCV, or by administering BCV at a high dose. This diagnosis may be a companion diagnosis for treatment with BCV, a pharmaceutically acceptable salt thereof, or a solvate thereof.

### (8) Method

An embodiment of the present invention provides a method for detecting an expression level of TLE1 from malignant tumor cells of a subject with a BCV-sensitive malignant tumor, comprising: (a) obtaining a sample from the subject (wherein the sample may be a malignant tumor cell, a malignant tumor cell lysate, or a solution containing nucleic acids or proteins contained in malignant tumor cells), or (b) detecting the TLE1 expression level of the sample after contacting the sample with a TLE1 detection reagent (including, for example, a nucleic acid or peptide described below). According to this method, the expression level of TLE1 from malignant tumor cells of a subject with a BCV-sensitive malignant tumor can be detected. For example, in malignant tumor cells with low TLE1 expression and high sensitivity to BCV, by detecting the low expression level of TLE1, treatment can be performed while anticipating that an effective therapeutic effect will be seen with BCV. For example, in malignant tumor cells with high TLE1 expression and high sensitivity to BCV, by detecting the high expression level of TLE1, treatment can be performed while anticipating that an effective therapeutic effect will be seen with BCV. For example, in malignant tumor cells with low TLE1 expression and high sensitivity to BCV, detecting the low expression level of TLE1 can be used as an indicator for setting the dose of BCV. For example, in malignant tumor cells with high TLE1 expression and high sensitivity to BCV, detecting the high expression level of TLE1 can be used as an indicator for setting the dose of BCV.

### (9) Method

An embodiment of the present invention provides a method for detecting an indicator for identifying a subject with a malignant tumor that has a high probability of being sensitive to BCV, a pharmaceutically acceptable salt thereof, or a solvate thereof, comprising testing an expression level of TLE1 in a sample derived from the subject with the malignant tumor. This method can be used to identify a subject with a malignant tumor that has a high probability of being sensitive to BCV, using the expression level of TLE1 as an indicator. Therefore, by utilizing this method, a more appropriate BCV treatment can be performed.

### (10) Method

An embodiment of the present invention provides a method for detecting an indicator for identifying a subject with a malignant tumor that has a high probability of responding or reacting to BCV, a pharmaceutically acceptable salt thereof, or a solvate thereof, comprising testing an expression level of TLE1 in a sample derived from the subject with the malignant tumor. This method can be used to identify a subject with a malignant tumor that has a high probability of responding or reacting to BCV, using the expression level of TLE1 as an indicator. Therefore, by utilizing this method, a more appropriate BCV treatment can be performed.

### (11) Method

An embodiment of the present invention provides a method for detecting a low expression level of TLE1 from malignant tumor cells of a subject to be administered BCV, a pharmaceutically acceptable salt thereof, or a solvate thereof, or from malignant tumor cells of a subject for whom administration thereof is being considered. This method comprises, for example: (a) obtaining a sample from the subject (wherein the sample may be a malignant tumor cell, a malignant tumor cell lysate, or a solution containing nucleic acids or proteins contained in malignant tumor cells), or (b) detecting the low expression level of TLE1 of the sample after contacting the sample with a TLE1 detection reagent (including, for example, a nucleic acid or peptide described below). According to this method, for example, in malignant tumor cells with low TLE1 expression and high sensitivity to BCV, by detecting the low expression level of TLE1, treatment can be performed while anticipating that an effective therapeutic effect will be seen with BCV. An embodiment of the present invention provides a method for detecting a high expression level of TLE1 from malignant tumor cells of a subject to be administered BCV, a pharmaceutically acceptable salt thereof, or a solvate thereof, or from malignant tumor cells of a subject for whom administration thereof is being considered. This method comprises, for example: (a) obtaining a sample from the subject (wherein the sample may be a malignant tumor cell, a malignant tumor cell lysate, or a solution containing nucleic acids or proteins contained in malignant tumor cells), or (b) detecting the high expression level of TLE1 of the sample after contacting the sample with a TLE1 detection reagent (including, for example, a nucleic acid or peptide described below). According to this method, for example, in malignant tumor cells with high TLE1 expression and high sensitivity to BCV, by detecting the high expression level of TLE1, treatment can be performed while anticipating that an effective therapeutic effect will be seen with BCV.

### (12) Method

An embodiment of the present invention provides a method for predicting the success of treatment with BCV, a pharmaceutically acceptable salt thereof, or a solvate thereof for a subject with a malignant tumor, comprising testing an expression level of TLE1 in a sample derived from the subject with the malignant tumor. This method can be used to identify a subject with a high probability of successful treatment with BCV, using the expression level of TLE1 as an indicator. Therefore, by utilizing this method, a more appropriate BCV treatment can be performed.

### (13) Method

An embodiment of the present invention provides a method for selecting a subject with a malignant tumor for whom a therapeutic effect of treatment with BCV, a pharmaceutically acceptable salt thereof, or a solvate thereof can be expected, comprising testing an expression level of TLE1 in a sample derived from the subject with the malignant tumor. This method can be used to select a subject for whom a therapeutic effect of treatment with BCV can be expected, using the expression level of TLE1 as an indicator. Therefore, by utilizing this method, a more appropriate BCV treatment can be performed.

### (14) Method

An embodiment of the present invention provides a method for obtaining an indicator for determining whether to perform a treatment method with BCV, a pharmaceutically acceptable salt thereof, or a solvate thereof, comprising testing an expression level of TLE1 in a sample derived from the subject with the malignant tumor. By utilizing this method, a more appropriate BCV treatment can be performed.

### (15) Method

An embodiment of the present invention provides a method for testing an expression level of TLE1 in a sample from a subject who has or is suspected of having a malignant tumor. This method may comprise, for example, measuring an expression level of TLE1 in a sample from the subject, or identifying a subject who provided a low TLE1 expression sample as a subject for administration of BCV, a pharmaceutically acceptable salt thereof, or a solvate thereof. By utilizing this method, a more appropriate BCV treatment can be performed by identifying a subject having a malignant tumor with low TLE1 expression and high sensitivity to BCV. This method may also comprise measuring an expression level of TLE1 in a sample from the subject, or identifying a subject who provided a high TLE1 expression sample or a high expression sample as a subject for administration of BCV, a pharmaceutically acceptable salt thereof, or a solvate thereof. By utilizing this method, a more appropriate BCV treatment can be performed by identifying a subject having a malignant tumor with high TLE1 expression and high sensitivity to BCV.

### (16) Method

An embodiment of the present invention provides a method for selecting a treatment method or an active ingredient used for treatment, comprising using an expression level of TLE1 as an indicator. This method may comprise, for example, testing an expression level of TLE1 in a sample derived from a subject with a malignant tumor. In this method, for example, if a subject's TLE1 has low expression and the subject is predicted to have high sensitivity to BCV, it may be selected to use BCV, a pharmaceutically acceptable salt thereof, or a solvate thereof for the treatment. In this method, for example, the dosage of BCV, a pharmaceutically acceptable salt thereof, or a solvate thereof may be adjusted or selected using the expression level of TLE1 as an indicator. The treatment method may comprise administering brincidofovir, a pharmaceutically acceptable salt thereof, or a solvate thereof to the subject. In this method, for example, if a subject's TLE1 has high expression and the subject is predicted to have high sensitivity to BCV, it may be selected to use BCV, a pharmaceutically acceptable salt thereof, or a solvate thereof for the treatment. This method may be a companion diagnostic method for selecting a treatment method or an active ingredient used for treatment.

### (17) Method

An embodiment of the present invention provides a method for predicting BCV sensitivity in tumor cell proliferation, comprising testing an expression level of TLE1 in a sample derived from a subject with a malignant tumor. By utilizing this method, tumor cell proliferation can be suppressed more effectively.

### (18) Method

An embodiment of the present invention provides a method for treating a malignant tumor, comprising administering BCV, a pharmaceutically acceptable salt thereof, or a solvate thereof to a subject having a malignant tumor. By utilizing this method, a malignant tumor can be treated. The treatment may be performed by administering a therapeutically effective amount of BCV, a pharmaceutically acceptable salt thereof, or a solvate thereof to the subject. The subject to be treated may be a subject having a lymphoma. The lymphoma may be an NK/T-cell lymphoma, a T-cell lymphoma, or a B-cell lymphoma. In the Examples described below, it was shown that the antitumor effect of BCV is particularly high for T-cell lymphoma. Therefore, T-cell lymphoma is particularly suitable as a therapeutic target for BCV.

### (19) Method

An embodiment of the present invention provides a method for predicting drug sensitivity or a method for determining a subject for drug administration, comprising testing an expression level of TLE1 in a sample derived from a subject with a malignant tumor. By utilizing this method, a malignant tumor can be treated more effectively.

### (20) Method

An embodiment of the present invention provides a method for predicting a prognosis of a malignant tumor, comprising using the expression level of TLE1 of a subject with a malignant tumor as an indicator. By utilizing this method, the prognosis of a patient can be predicted. For example, if the expression level of TLE1 in a sample from a subject with a malignant tumor (e.g., an NK/T-cell lymphoma or a T-cell lymphoma) is low, a poor prognosis may be predicted. For example, if the expression level of TLE1 in a sample from a subject with a malignant tumor (e.g., an NK/T-cell lymphoma or a T-cell lymphoma) is high, a good prognosis may be predicted. This prediction may further predict the prognosis based on the expression level of MYC of the subject with the malignant tumor. For example, if the expression level of TLE1 in a sample from a subject with a malignant tumor (e.g., an NK/T-cell lymphoma or a T-cell lymphoma) is low, and the subject is MYC-positive, a poor prognosis may be evaluated. For example, if the expression level of TLE1 in a sample from a subject with a malignant tumor (e.g., an NK/T-cell lymphoma or a T-cell lymphoma) is high, and the subject is MYC-negative, a good prognosis may be evaluated. If the malignant tumor is a B-cell lymphoma, for example, a poor prognosis may be predicted if the expression level of TLE1 in a sample from the subject is high, and a good prognosis may be predicted if the expression level of TLE1 in a sample from the subject is low. This prediction may further predict the prognosis based on the expression level of MYC of the subject with the malignant tumor. For example, if the expression level of TLE1 in a sample from a subject with a B-cell lymphoma is high, and the subject is MYC-positive, a poor prognosis may be evaluated. For example, if the expression level of TLE1 in a sample from a subject with a B-cell lymphoma is low, and the subject is MYC-negative, a good prognosis may be evaluated.

### (21) Method

According to an embodiment of the present invention, in the methods described in (1) to (20) above, ZNF441 may be used as an indicator or a subject of testing, instead of or in addition to using TLE1 as an indicator or a subject of testing. In this case, in the descriptions of (1) to (20) above, "if the expression level of TLE1 is low" can be read as "if the expression level of ZNF441 is high"; "a subject's TLE1 expression level is low" can be read as "a subject's ZNF441 expression level is high"; "a subject's TLE1 expression level is high" can be read as "a subject's ZNF441 expression level is low"; "low TLE1 expression" can be read as "high ZNF441 expression"; "expression level of TLE1" can be read as "expression level of ZNF441"; "expression level of TLE1 is low" can be read as "expression level of ZNF441 is high"; and "expression level of TLE1 is high" can be read as "expression level of ZNF441 is low". The target disease when ZNF441 is used as an indicator or a subject of testing may be an NK/T-cell lymphoma. Furthermore, according to an embodiment of the present invention, in the methods described in (1) to (20) above, any one or more proteins or genes whose names are described in Fig. 3 may be used as an indicator, instead of or in addition to using TLE1 or ZNF441 as an indicator or a subject of testing. In this case, the embodiments of FAM89A and AK4 in Fig. 3 may adopt the same embodiments as TLE1 (e.g., (1) to (20) above) (in this case, TLE1 may be read as FAM89A or AK4), and proteins or genes other than FAM89A and AK4 may adopt the same embodiments as ZNF441 (in this case, ZNF441 may be read as the name of each protein or gene).

### (22) Method

According to an embodiment of the present invention, in the methods described in (1) to (20) above, Ceramide synthase 6 (CERS6) may be used as an indicator or a subject of testing, instead of or in addition to using TLE1 as an indicator or a subject of testing. In this case, in the descriptions of (1) to (20) above, "if the expression level of TLE1 is low" can be read as "if the expression level of CERS6 is high"; "a subject's TLE1 expression level is low" can be read as "a subject's CERS6 expression level is high"; "a subject's TLE1 expression level is high" can be read as "a subject's CERS6 expression level is low"; "low TLE1 expression" can be read as "high CERS6 expression"; "expression level of TLE1" can be read as "expression level of CERS6"; "expression level of TLE1 is low" can be read as "expression level of CERS6 is high"; and "expression level of TLE1 is high" can be read as "expression level of CERS6 is low". The target disease when CERS6 is used as an indicator or a subject of testing may be a B-cell lymphoma. Furthermore, according to an embodiment of the present invention, in the methods described in (1) to (20) above, any one or more proteins or genes whose names are described in Fig. 16 may be used as an indicator, instead of or in addition to using TLE1 or CERS6 as an indicator or a subject of testing. The embodiments of these proteins or genes may adopt the same embodiments as TLE1 or CERS6 (e.g., (1) to (21) above) (in this case, TLE1 or CERS6 may be read as the name of each protein or gene).

### (23) Method

An embodiment of the present invention provides a method for treating a malignant tumor, comprising administering BCV, a pharmaceutically acceptable salt thereof, or a solvate thereof to a subject having a malignant tumor, wherein the subject is a subject identified based on an expression level of ZNF441 as an indicator. As described in the Examples below, BCV sensitivity in malignant tumors was associated with the expression level of ZNF441. For example, it was shown that when the expression level of ZNF441 is high, the inhibitory effect by BCV is high, and the sensitivity is high. Therefore, by utilizing this method, sensitivity can be predicted using the expression level of ZNF441 as an indicator, and a more appropriate BCV treatment can be performed. For example, if a subject's ZNF441 expression level is high and the subject is predicted to have high sensitivity, treatment may be performed by administering BCV, or by administering BCV at a low dose. On the other hand, for example, if a subject's ZNF441 expression level is low and the subject is predicted to have low sensitivity, treatment may be performed by not administering BCV, or by administering BCV at a high dose.

### (24) Method

An embodiment of the present invention provides a method for treating a malignant tumor, comprising administering BCV, a pharmaceutically acceptable salt thereof, or a solvate thereof to a subject having a malignant tumor, wherein the subject is a subject identified based on an expression level of CERS6 as an indicator. As described in the Examples below, BCV sensitivity in malignant tumors was associated with the expression level of CERS6. For example, it was shown that when the expression level of CERS6 is high, the inhibitory effect by BCV is high, and the sensitivity is high. Therefore, by utilizing this method, sensitivity can be predicted using the expression level of CERS6 as an indicator, and a more appropriate BCV treatment can be performed. For example, if a subject's CERS6 expression level is high and the subject is predicted to have high sensitivity, treatment may be performed by administering BCV, or by administering BCV at a low dose. On the other hand, for example, if a subject's CERS6 expression level is low and the subject is predicted to have low sensitivity, treatment may be performed by not administering BCV, or by administering BCV at a high dose.

### (25) Method

An embodiment of the present invention provides a method for predicting sensitivity to BCV, a pharmaceutically acceptable salt thereof, or a solvate thereof in a subject with a malignant tumor, comprising testing an expression level of ZNF441 in a sample derived from the subject with the malignant tumor. This method can be used to predict or identify a subject with high sensitivity or low sensitivity to BCV using the expression level of ZNF441 as an indicator. Therefore, by utilizing this method, a more appropriate BCV treatment can be performed. For example, if a subject's ZNF441 expression level is high and the subject is predicted to have high sensitivity, treatment may be performed by administering BCV, or by administering BCV at a low dose. On the other hand, for example, if a subject's ZNF441 expression level is low and the subject is predicted to have low sensitivity, treatment may be performed by not administering BCV, or by administering BCV at a high dose. From another aspect, an embodiment of the present invention provides a method for predicting sensitivity to BCV, a pharmaceutically acceptable salt thereof, or a solvate thereof in a subject with a malignant tumor, comprising testing an expression level of ZNF441 in malignant tumor cells derived from the subject with the malignant tumor.

### (26) Method

An embodiment of the present invention provides a method for predicting sensitivity to BCV, a pharmaceutically acceptable salt thereof, or a solvate thereof in a subject with a malignant tumor, comprising testing an expression level of CERS6 in a sample derived from the subject with the malignant tumor. This method can be used to predict or identify a subject with high sensitivity or low sensitivity to BCV using the expression level of CERS6 as an indicator. Therefore, by utilizing this method, a more appropriate BCV treatment can be performed. For example, if a subject's CERS6 expression level is high and the subject is predicted to have high sensitivity, treatment may be performed by administering BCV, or by administering BCV at a low dose. On the other hand, for example, if a subject's CERS6 expression level is low and the subject is predicted to have low sensitivity, treatment may be performed by not administering BCV, or by administering BCV at a high dose. From another aspect, an embodiment of the present invention provides a method for predicting sensitivity to BCV, a pharmaceutically acceptable salt thereof, or a solvate thereof in a subject with a malignant tumor, comprising testing an expression level of CERS6 in malignant tumor cells derived from the subject with the malignant tumor.

### (27) Method

An embodiment of the present invention provides a method for detecting an expression level of ZNF441 from malignant tumor cells of a subject with a BCV-sensitive malignant tumor. This method may comprise, for example: (a) obtaining a sample from the subject (wherein the sample may be a malignant tumor cell, a malignant tumor cell lysate, or a solution containing nucleic acids or proteins contained in malignant tumor cells), or (b) detecting the ZNF441 expression level of the sample after contacting the sample with a ZNF441 detection reagent (including, for example, a nucleic acid or peptide described below). According to this method, the expression level of ZNF441 from malignant tumor cells of a subject with a BCV-sensitive malignant tumor can be detected. For example, in malignant tumor cells with high ZNF441 expression and high sensitivity to BCV, by detecting the high expression level of ZNF441, treatment can be performed while anticipating that an effective therapeutic effect will be seen with BCV. For example, in malignant tumor cells with high ZNF441 expression and high sensitivity to BCV, detecting the high expression level of ZNF441 can be used as an indicator for setting the dose of BCV.

### (28) Method

An embodiment of the present invention provides a method for detecting an expression level of CERS6 from malignant tumor cells of a subject with a BCV-sensitive malignant tumor. This method may comprise, for example: (a) obtaining a sample from the subject (wherein the sample may be a malignant tumor cell, a malignant tumor cell lysate, or a solution containing nucleic acids or proteins contained in malignant tumor cells), or (b) detecting the CERS6 expression level of the sample after contacting the sample with a CERS6 detection reagent (including, for example, a nucleic acid or peptide described below). According to this method, the expression level of CERS6 from malignant tumor cells of a subject with a BCV-sensitive malignant tumor can be detected. For example, in malignant tumor cells with high CERS6 expression and high sensitivity to BCV, by detecting the high expression level of CERS6, treatment can be performed while anticipating that an effective therapeutic effect will be seen with BCV. For example, in malignant tumor cells with high CERS6 expression and high sensitivity to BCV, detecting the high expression level of CERS6 can be used as an indicator for setting the dose of BCV.

### (29) Method

The method of the embodiments of the present invention (e.g., (1) to (28) above) may comprise, for example: (i) testing whether a subject has a malignant tumor, (ii) identifying a subject having a malignant tumor, (iii) identifying a subject who is positive for a malignant tumor marker, (iv) collecting, obtaining, or preparing a sample of the subject (e.g., a malignant tumor cell or a cell lysate thereof), (v) collecting malignant tumor cells from the subject, (vi) extracting a nucleic acid (e.g., genomic DNA or total RNA) from the subject's sample, (vii) testing an expression level of TLE1 in the subject or a sample of the subject, (viii) performing the intended method using an expression level of TLE1 in the subject or a sample of the subject as an indicator, (ix) identifying the subject as a subject for treatment with BCV using an expression level of TLE1 in the subject or a sample of the subject as an indicator, (x) determining a subject with a malignant tumor to be administered BCV using an expression level of TLE1 in the subject or a sample of the subject as an indicator, (xi) predicting the subject's sensitivity to BCV using an expression level of TLE1 in the subject or a sample of the subject as an indicator, (xii) diagnosing whether the subject is a subject having a malignant tumor with high sensitivity to BCV using an expression level of TLE1 in the subject or a sample of the subject as an indicator, (xiii) identifying the subject as a subject for treatment with BCV if the TLE1 expression level in the subject or a sample of the subject is low, or identifying the subject as a subject for treatment with BCV if the TLE1 expression level in the subject or a sample of the subject is high, (xiv) identifying a subject having a malignant tumor with low TLE1 expression as a subject to be treated, or identifying a subject having a malignant tumor with high TLE1 expression as a subject to be treated, (xv) identifying a subject having a malignant tumor with low TLE1 expression as a subject for treatment with low-dose BCV, or identifying a subject having a malignant tumor with low TLE1 expression or a malignant tumor with high TLE1 expression as a subject for treatment with low-dose BCV, (xvi) identifying a subject having a malignant tumor with high TLE1 expression as a subject for treatment with high-dose BCV, or identifying a subject having a malignant tumor with low TLE1 expression as a subject for treatment with high-dose BCV, (xvii) administering BCV if the TLE1 expression level in the subject or a sample of the subject is low, or administering BCV if the TLE1 expression level in the subject or a sample of the subject is high, (xviii) administering BCV at a low dose if the TLE1 expression level in the subject or a sample of the subject is low, or administering BCV at a low dose if the TLE1 expression level in the subject or a sample of the subject is high, (xix) not administering BCV if the TLE1 expression level in the subject or a sample of the subject is high, or not administering BCV if the TLE1 expression level in the subject or a sample of the subject is low, (xx) administering BCV at a high dose if the TLE1 expression level in the subject or a sample of the subject is high, or administering BCV at a high dose if the TLE1 expression level in the subject or a sample of the subject is low, (xxi) indicating that the subject is sensitive to BCV (or has a high probability thereof) if the TLE1 expression level in the subject or a sample of the subject is low, or indicating that the subject is sensitive to BCV (or has a high probability thereof) if the TLE1 expression level in the subject or a sample of the subject is high, (xxii) indicating that the subject is not sensitive to BCV (or has a high probability thereof) if the TLE1 expression level in the subject or a sample of the subject is high, or indicating that the subject is not sensitive to BCV (or has a high probability thereof) if the TLE1 expression level in the subject or a sample of the subject is low, (xxiii) testing an expression level of ZNF441 or CERS6 in the subject or a sample of the subject, (xxiv) performing the intended method using an expression level of ZNF441 or CERS6 in the subject or a sample of the subject as an indicator, (xxv) identifying the subject as a subject for treatment with BCV using an expression level of ZNF441 or CERS6 in the subject or a sample of the subject as an indicator, (xxvi) determining a subject with a malignant tumor to be administered BCV using an expression level of ZNF441 or CERS6 in the subject or a sample of the subject as an indicator, (xxvii) predicting the subject's sensitivity to BCV using an expression level of ZNF441 or CERS6 in the subject or a sample of the subject as an indicator, (xxviii) diagnosing whether the subject is a subject having a malignant tumor with high sensitivity to BCV using an expression level of ZNF441 or CERS6 in the subject or a sample of the subject as an indicator, (xxix) identifying the subject as a subject for treatment with BCV if the expression level of ZNF441 or CERS6 in the subject or a sample of the subject is high, (xxx) identifying a subject having a malignant tumor with high ZNF441 or CERS6 expression as a subject to be treated, (xxxi) identifying a subject having a malignant tumor with high ZNF441 or CERS6 expression as a subject for treatment with low-dose BCV, (xxxii) identifying a subject having a malignant tumor with low ZNF441 or CERS6 expression as a subject for treatment with high-dose BCV, (xxxiii) administering BCV if the expression level of ZNF441 or CERS6 in the subject or a sample of the subject is high, (xxxiv) administering BCV at a low dose if the expression level of ZNF441 or CERS6 in the subject or a sample of the subject is high, (xxxv) not administering BCV if the expression level of ZNF441 or CERS6 in the subject or a sample of the subject is low, (xxxvi) administering BCV at a high dose if the expression level of ZNF441 or CERS6 in the subject or a sample of the subject is low, (xxxvii) indicating that the subject is sensitive to BCV (or has a high probability thereof) if the expression level of ZNF441 or CERS6 in the subject or a sample of the subject is high, (xxxviii) indicating that the subject is not sensitive to BCV (or has a high probability thereof) if the expression level of ZNF441 or CERS6 in the subject or a sample of the subject is low, (xxxix) testing for the presence or absence of MYC positivity in the subject or a sample of the subject, (xxxx) identifying the subject as a subject for treatment with BCV using the presence or absence of MYC positivity in the subject or a sample of the subject as an indicator, (xxxxi) administering BCV to the subject if the subject or a sample of the subject is MYC-positive, (xxxxii) predicting a prognosis of the malignant tumor using an expression level of MYC in the subject or a sample of the subject as an indicator, (xxxxiii) administering a therapeutically effective amount of BCV to the subject, (xxxxiv) suppressing the proliferation of malignant tumor cells of the subject, (xxxxv) reducing a malignant tumor marker in the subject, or (xxxxvi) reducing the malignant tumor size of the subject. The method of the embodiments of the present invention (e.g., (1) to (28) above) can adopt any one or more of these steps (i) to (xxxxvi), which is useful for a more appropriate BCV treatment (e.g., for administering treatment to a population in which the treatment is highly effective). When two or more are adopted, the order is arbitrary and can be determined according to the desired treatment method. Any one or more of these steps (i) to (xxxxvi) may be performed before or after the step of administering BCV, a pharmaceutically acceptable salt thereof, or a solvate thereof to the subject. In addition, the method of the embodiments of the present invention (e.g., (1) to (28) above) may or may not comprise, for example, when specified: identifying a subject having a cytomegalovirus (CMV), adenovirus (AdV), BK virus (BKV), or variola virus (VaV) infection, identifying a CMV, AdV, BKV, or VaV seropositive subject, identifying a subject in need of prevention or treatment of a CMV, AdV, BKV, or VaV infection, identifying a subject after allogeneic transplantation (e.g., hematopoietic cell transplantation), identifying a subject in need of an immunosuppressant, identifying a subject in an immunosuppressed state, a method for the purpose of treating a virus-induced tumor in a subject in an immunosuppressed state, or a method for the purpose of preventing or treating a viral (e.g., CMV, AdV, BKV, or VaV) infection.

### (30) Composition

An embodiment of the present invention provides a pharmaceutical composition for treatment of a malignant tumor, comprising BCV, a pharmaceutically acceptable salt thereof, or a solvate thereof. The subject of the treatment may be a subject having a malignant tumor with low TLE1 expression, or a subject identified based on an expression level of TLE1 as an indicator. In the Examples described below, BCV sensitivity in malignant tumors was associated with the expression level of TLE1. For example, it was shown that NK/T-cell lymphomas or T-cell lymphomas with a low expression level of TLE1 have high sensitivity. By utilizing this pharmaceutical composition, it is possible to administer BCV to a subject who is expected to have low TLE1 expression and high sensitivity to BCV, and in that case, an improvement of the therapeutic effect or an increase in the probability of a therapeutic effect appearing can be expected, as compared to when BCV is administered to an unspecified subject. In addition, this pharmaceutical composition can be used to predict sensitivity using the expression level of TLE1 as an indicator and to perform a more appropriate BCV treatment. For example, if a subject's TLE1 expression level is low and the subject is predicted to have high sensitivity, treatment may be performed by administering BCV, or by administering BCV at a low dose. Also, for example, if a subject's TLE1 expression level is high and the subject is predicted to have low sensitivity, treatment may be performed by not administering BCV, or by administering BCV at a high dose. In addition, as described in the Examples below, the prognosis was particularly poor in patients with BCV-untreated NK/T-cell lymphoma with low TLE1 expression. Therefore, this pharmaceutical composition can be used for the treatment of malignant tumors with a poor prognosis. In addition, in the Examples described below, it was shown that B-cell lymphomas with a high expression level of TLE1 have high sensitivity. Therefore, by utilizing this pharmaceutical composition, it is possible to administer BCV to a subject who is expected to have high TLE1 expression and high sensitivity to BCV, and in that case, an improvement of the therapeutic effect or an increase in the probability of a therapeutic effect appearing can be expected, as compared to when BCV is administered to an unspecified subject. In addition, the pharmaceutical composition can be used to predict sensitivity using the expression level of TLE1 as an indicator and to perform a more appropriate BCV treatment. For example, if a subject's TLE1 expression level is high and the subject is predicted to have high sensitivity, treatment may be performed by administering BCV, or by administering BCV at a low dose. Also, for example, if a subject's TLE1 expression level is low and the subject is predicted to have low sensitivity, treatment may be performed by not administering BCV, or by administering BCV at a high dose. In addition, as described in the Examples below, the prognosis was particularly poor in patients with BCV-untreated B-cell lymphoma with high TLE1 expression. Therefore, this pharmaceutical composition can be used for the treatment of malignant tumors with a poor prognosis.

### (31) Composition

An embodiment of the present invention provides a composition for use in predicting sensitivity to BCV, a pharmaceutically acceptable salt thereof, or a solvate thereof in a subject with a malignant tumor, the composition comprising a test reagent for an expression level of TLE1. This composition can be used to predict or identify a subject with high sensitivity or low sensitivity to BCV, using the expression level of TLE1 as an indicator. Therefore, this composition can be used to perform a more appropriate BCV treatment. For example, if a subject's TLE1 expression level is low and the subject is predicted to have high sensitivity, treatment may be performed by administering BCV, or by administering BCV at a low dose. For example, if a subject's TLE1 expression level is high and the subject is predicted to have low sensitivity, treatment may be performed by not administering BCV, or by administering BCV at a high dose. For example, if a subject's TLE1 expression level is high and the subject is predicted to have high sensitivity, treatment may be performed by administering BCV, or by administering BCV at a low dose. For example, if a subject's TLE1 expression level is low and the subject is predicted to have low sensitivity, treatment may be performed by not administering BCV, or by administering BCV at a high dose.

### (32) Composition

An embodiment of the present invention provides a composition for use in determining a subject with a malignant tumor to be administered BCV, a pharmaceutically acceptable salt thereof, or a solvate thereof, the composition comprising a test reagent for an expression level of TLE1. This composition can be used to perform a more appropriate BCV treatment. For example, if a subject's TLE1 expression level is low and the subject is predicted to have high sensitivity, the subject may be determined as a subject for administration of BCV or as a subject for administration at a low dose, and if a subject's TLE1 expression level is high and the subject is predicted to have low sensitivity, the subject may be determined as a subject for administration of BCV at a high dose. For example, if a subject's TLE1 expression level is high and the subject is predicted to have high sensitivity, the subject may be determined as a subject for administration of BCV or as a subject for administration at a low dose, and if a subject's TLE1 expression level is low and the subject is predicted to have low sensitivity, the subject may be determined as a subject for administration of BCV at a high dose. The subject to be administered may be a subject who should be administered. From another aspect, an embodiment of the present invention provides a composition for use in selecting a subject with a malignant tumor who is suitable for treatment with BCV, a pharmaceutically acceptable salt thereof, or a solvate thereof, the composition comprising a test reagent for an expression level of TLE1.

### (33) Composition

An embodiment of the present invention provides a composition for use in diagnosing whether a subject is a subject having a malignant tumor with high sensitivity to brincidofovir, a pharmaceutically acceptable salt thereof, or a solvate thereof, the composition comprising a test reagent for an expression level of TLE1. This composition can be used to diagnose whether a subject is a subject having a malignant tumor with high sensitivity to BCV, using the expression level of TLE1 as an indicator. Therefore, this composition can be used to perform a more appropriate BCV treatment. For example, if a subject is diagnosed as being a subject having a malignant tumor with high sensitivity, treatment may be performed by administering BCV, or by administering BCV at a low dose. On the other hand, for example, if a subject is diagnosed as not being a subject having a malignant tumor with high sensitivity, treatment may be performed by not administering BCV, or by administering BCV at a high dose. This diagnosis may be a companion diagnosis for treatment with BCV, a pharmaceutically acceptable salt thereof, or a solvate thereof.

### (34) Composition

An embodiment of the present invention provides a composition for use in detecting an indicator for identifying a subject with a malignant tumor that has a high probability of being sensitive to BCV, a pharmaceutically acceptable salt thereof, or a solvate thereof, the composition comprising a test reagent for an expression level of TLE1. From another aspect, an embodiment of the present invention provides a composition for use in detecting an indicator for identifying a subject with a malignant tumor that has a high probability of responding or reacting to BCV, a pharmaceutically acceptable salt thereof, or a solvate thereof, the composition comprising a test reagent for an expression level of TLE1. From another aspect, an embodiment of the present invention provides a composition for use in detecting an expression level of TLE1 in a sample from a subject who has or is suspected of having a malignant tumor, the composition comprising a test reagent for an expression level of TLE1. By using any of these methods, a more appropriate BCV treatment can be performed.

### (35) Composition

An embodiment of the present invention provides a pharmaceutical composition for treatment of a malignant tumor, comprising BCV, a pharmaceutically acceptable salt thereof, or a solvate thereof. By utilizing this pharmaceutical composition, a malignant tumor can be treated. The pharmaceutical composition may comprise BCV, a pharmaceutically acceptable salt thereof, or a solvate thereof as an active ingredient. The malignant tumor may be a lymphoma. The lymphoma may be an NK/T-cell lymphoma, a T-cell lymphoma, or a B-cell lymphoma. In the Examples described below, it was shown that the antitumor effect of BCV is particularly high for T-cell lymphoma. Therefore, T-cell lymphoma is particularly suitable as a therapeutic target for BCV.

### (36) Composition

An embodiment of the present invention provides a composition for use in predicting a prognosis of a malignant tumor, the composition comprising a test reagent for an expression level of TLE1. By utilizing this composition, the prognosis of a patient can be predicted. This composition may be used to predict a prognosis based on the expression level of TLE1 of a subject with a malignant tumor. For example, if the expression level of TLE1 in a sample from a subject with a malignant tumor (e.g., an NK/T-cell lymphoma or a T-cell lymphoma) is low, a poor prognosis may be evaluated. For example, if the expression level of TLE1 in a sample from a subject with a malignant tumor (e.g., an NK/T-cell lymphoma or a T-cell lymphoma) is high, a good prognosis may be evaluated. This prediction may further predict the prognosis based on the expression level of MYC of the subject with the malignant tumor. For example, if the expression level of TLE1 in a sample from a subject with a malignant tumor (e.g., an NK/T-cell lymphoma or a T-cell lymphoma) is low, and the subject is MYC-positive, a poor prognosis may be evaluated. For example, if the expression level of TLE1 in a sample from a subject with a malignant tumor (e.g., an NK/T-cell lymphoma or a T-cell lymphoma) is high, and the subject is MYC-negative, a good prognosis may be evaluated. The expression level of MYC may be tested using a test reagent for MYC. If the malignant tumor is a B-cell lymphoma, for example, a poor prognosis may be predicted if the expression level of TLE1 in a sample from the subject is high, and a good prognosis may be predicted if the expression level of TLE1 in a sample from the subject is low. This prediction may further predict the prognosis based on the expression level of MYC of the subject with the malignant tumor. For example, if the expression level of TLE1 in a sample from a subject with a B-cell lymphoma is high, and the subject is MYC-positive, a poor prognosis may be evaluated. For example, if the expression level of TLE1 in a sample from a subject with a B-cell lymphoma is low, and the subject is MYC-negative, a good prognosis may be evaluated.

### (37) Composition

An embodiment of the present invention provides a composition for use in the methods described in (1) to (29) above, comprising a test reagent for an expression level of TLE1, ZNF441, CERS6, or a protein or gene described in Fig. 3 or 16.

### (38) Composition

An embodiment of the present invention provides a composition for use in the methods described in (1) to (29) above, comprising BCV, a pharmaceutically acceptable salt thereof, or a solvate thereof.

### (39) Kit

An embodiment of the present invention provides a kit for use in the methods described in (1) to (29) above, comprising a test reagent for an expression level of TLE1, ZNF441, CERS6, or a protein or gene described in Fig. 3 or 16. The kit may comprise, for example, instructions for use, a buffer solution, a container, or packaging.

### (40) Use

An embodiment of the present invention provides a use of TLE1, ZNF441, CERS6, or a protein or gene described in Fig. 3 or 16 as a biomarker for use in the methods described in (1) to (29) above.

### (41) Use

An embodiment of the present invention provides a use of BCV, a pharmaceutically acceptable salt thereof, or a solvate thereof, for the manufacture of the compositions described in (30) to (38) above.

In embodiments of the present invention (e.g., (1) to (41) above), brincidofovir (BCV) includes a compound having a structure represented by the following formula. BCV may also be represented by the IUPAC name of [(2S)-1-(4-amino-2-oxopyrimidin-1-yl)-3-hydroxypropan-2-yl]oxymethyl-(3-hexadecoxypropoxy)phosphinic acid. BCV includes a compound represented by CAS Registry Number 444805-28-1. In the present description, BCV is an abbreviation for brincidofovir, and they have the same meaning.

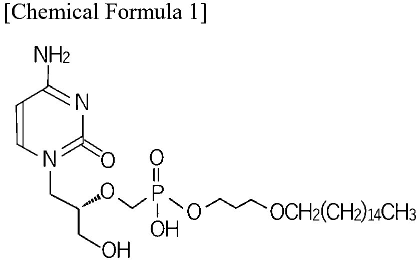

In embodiments of the present invention (e.g., (1) to (41) above), TLE1 includes a protein represented by Transducin-like enhancer of split 1 or TLE family member 1, transcriptional corepressor. Details such as the amino acid sequence of TLE1 can be confirmed from websites such as NCBI or UniProt. As of the filing of the present application, the Primary accession number of TLE1 in UniProt is Q04724, the HGNC ID is HGNC:11837, and the Gene ID in NCBI is 7088 (updated on 9-Apr-2023). In the Examples described below, TLE1 corresponding to the above Q04724 was detected. TLE1 may be, for example, TLE1 derived from a human, monkey, mouse, rat, rabbit, dog, or cat.

In embodiments of the present invention (e.g., (1) to (41) above), ZNF441 includes a protein represented by Zinc finger protein 441. Details such as the amino acid sequence of ZNF441 can be confirmed from websites such as NCBI or UniProt. As of the filing of the present application, the Primary accession number of ZNF441 in UniProt is Q8N8Z8, the HGNC ID is HGNC:20875, and the Gene ID in NCBI is 126068. In the Examples described below, ZNF441 corresponding to the above Q8N8Z8 was detected. ZNF441 may be, for example, ZNF441 derived from a human, monkey, mouse, rat, rabbit, dog, or cat. In embodiments of the present invention (e.g., (1) to (41) above), CERS6 includes a protein represented by Ceramide synthase 6 or LASS6. Details such as the amino acid sequence of CERS6 can be confirmed from websites such as NCBI or UniProt. As of the filing of the present application, the Primary accession number of CERS6 in UniProt is Q6ZMG9, the HGNC ID is HGNC:23826, and the Gene ID in NCBI is 253782. In the Examples described below, CERS6 corresponding to the above Q6ZMG9 was detected. CERS6 may be, for example, CERS6 derived from a human, monkey, mouse, rat, rabbit, dog, or cat.

In embodiments of the present invention (e.g., (1) to (41) above), a malignant tumor includes, for example, a tumor that arises when normal cells undergo mutation. Malignant tumors can arise from any organ or tissue in the entire body. This malignant tumor includes, for example, one or more selected from the group consisting of a hematologic malignancy (e.g., lymphoma, leukemia or multiple myeloma), lung cancer, esophageal cancer, stomach cancer, liver cancer, pancreatic cancer, kidney cancer, adrenal cancer, biliary tract cancer, breast cancer, colorectal cancer, small intestine cancer, ovarian cancer, uterine cancer, bladder cancer, prostate cancer, ureteral cancer, renal pelvis cancer, penile cancer, testicular cancer, a brain tumor (e.g., glioma), cancer of the central nervous system, cancer of the peripheral nervous system, head and neck cancer, skin cancer (e.g., melanoma), thyroid cancer, salivary gland cancer, carcinoma, and sarcoma. The hematologic malignancy may be a hematologic cancer. The above-described leukemia includes, for example, myeloid leukemia (e.g., acute myeloid leukemia or chronic myeloid leukemia) and lymphoid leukemia (e.g., acute lymphoid leukemia or chronic lymphoid leukemia). The therapeutic effect on a malignant tumor may be evaluated, for example, by observing the change over time in the proliferation of malignant tumor cells after drug administration. A state in which cell proliferation is suppressed includes a state in which the proliferation rate of the test cells is significantly decreased compared to that before drug treatment. The proliferation rate may be measured, for example, using absorbance as an indicator, or may be determined from image data. The therapeutic effect on a malignant tumor may be evaluated, for example, by observing a reduction in tumor size or tumor mass after drug administration. At this time, when the tumor size or tumor mass is significantly reduced compared to that before drug administration or upon administration of a negative control, a therapeutic effect may be considered present. The therapeutic effect on a malignant tumor may be measured, for example, using the amount of a malignant tumor marker in a patient or a patient-derived sample as an indicator. At this time, when the marker amount is significantly reduced compared to that before drug administration or upon administration of a negative control, a therapeutic effect may be considered present. The tumor mass or marker amount after drug administration may be reduced to 0.9, 0.7, 0.5, 0.3, or 0.1 times or less compared to that before drug administration or upon administration of a negative control. The malignant tumor may be a malignant tumor with low TLE1 expression. A malignant tumor with low TLE1 expression may include a TLE1-negative malignant tumor. The malignant tumor may be a malignant tumor with high TLE1 expression. A malignant tumor with high TLE1 expression may include a TLE1-positive malignant tumor. The malignant tumor may be a malignant tumor with high expression of ZNF441, CERS6, a protein or gene other than FAM89A and AK4 in Fig. 3, or a protein or gene described in Fig. 16. This malignant tumor with high expression may include a positive malignant tumor.

In embodiments of the present invention (e.g., (1) to (41) above), when the malignant tumor is a hematologic cancer, the hematologic cancer may include, for example, a lymphoma, a leukemia, or a multiple myeloma. The leukemia may include, for example, acute myeloid leukemia, acute lymphoid leukemia, chronic myeloid leukemia, or chronic lymphoid leukemia. When the malignant tumor is a lymphoma, the lymphoma includes a malignant lymphoma. Malignant lymphoma includes, for example, a disease caused by cancerous changes in lymphocytes. Malignant lymphoma includes, for example, non-Hodgkin's lymphoma or Hodgkin's lymphoma. Non-Hodgkin's lymphoma includes, for example, B-cell lymphoma, NK/T-cell lymphoma, or T-cell lymphoma. B-cell lymphoma includes, for example, a B-cell lymphoma classified into one or more of follicular lymphoma, MALT lymphoma, lymphoplasmacytic lymphoma, mantle cell lymphoma, diffuse large B-cell lymphoma (DLBCL), Burkitt's lymphoma, primary effusion lymphoma, or chronic lymphocytic leukemia/small lymphocytic lymphoma. NK/T-cell lymphoma includes, for example, an NK/T-cell lymphoma classified into one or more of peripheral T-cell lymphoma, angioimmunoblastic T-cell lymphoma, anaplastic large cell lymphoma, adult T-cell leukemia-lymphoma, extranodal NK/T-cell lymphoma, nasal type, or cutaneous lymphoma (e.g., mycosis fungoides). T-cell lymphoma includes a T-cell lymphoma classified into one or more of peripheral T-cell lymphoma (PTCL), cutaneous T-cell lymphoma, anaplastic large cell lymphoma, enteropathy-associated T-cell lymphoma, or lymphoblastic lymphoma. Non-Hodgkin's lymphoma includes, for example, peripheral T-cell lymphoma. Peripheral T-cell lymphoma includes, for example, a peripheral T-cell lymphoma classified into one or more of nodal peripheral T-cell lymphoma or extranodal peripheral T-cell lymphoma. Nodal peripheral T-cell lymphoma includes, for example, a nodal peripheral T-cell lymphoma classified into one or more of PTCL, not otherwise specified (PTCL-NOS), PTCL with a follicular helper T-cell phenotype, or anaplastic large cell lymphoma, ALK positive. Extranodal peripheral T-cell lymphoma includes, for example, an extranodal peripheral T-cell lymphoma classified as extranodal NK/T-cell lymphoma, nasal type. From the viewpoint of improving the therapeutic effect or increasing the probability of a therapeutic effect appearing, the T-cell lymphoma is preferably a peripheral T-cell lymphoma. Although peripheral T-cell lymphoma is generally a difficult disease to treat, in the Examples described below, a therapeutic effect by BCV was observed even for peripheral T-cell lymphoma cell lines. Hodgkin's lymphoma includes, for example, classical Hodgkin's lymphoma or nodular lymphocyte-predominant Hodgkin's lymphoma. For details of lymphoma, reference may be made to, for example, Nirmal, J Oral Maxillofac Pathol. 2020 May-Aug;24(2):195-199, Singh et al., J Family Med Prim Care. 2020 Apr; 9(4): 1834-1840, Voltin et al., Cancers (Basel). 2020 Mar 5;12(3):601, or Pu et al., Front Immunol. 2022; 13: 1008695. From the viewpoint of improving the therapeutic effect or increasing the probability of a therapeutic effect appearing, the lymphoma is preferably an NK/T-cell lymphoma, a T-cell lymphoma, or a B-cell lymphoma. The therapeutic effect on or onset of lymphoma may be diagnosed by examination of lymph node swelling with CT, PET, or MRI, a histological examination of a lymph node or a tumor mass, or an examination of a tumor marker for malignant lymphoma in blood (e.g., sIL2-R). The diagnosis of lymphoma may be performed, for example, by the methods described in Nirmal (supra), Voltin et al. (supra), or Pu et al. (supra).

In embodiments of the present invention (e.g., (1) to (41) above), a malignant tumor with low TLE1 expression and high sensitivity to BCV includes an NK/T-cell lymphoma or a T-cell lymphoma with low TLE1 expression. A malignant tumor with high TLE1 expression and high sensitivity to BCV includes a B-cell lymphoma with high TLE1 expression. A malignant tumor with high ZNF441 expression and high sensitivity to BCV includes an NK/T-cell lymphoma with high ZNF441 expression. A malignant tumor with high CERS6 expression and high sensitivity to BCV includes a B-cell lymphoma with high CERS6 expression.

In embodiments of the present invention (e.g., (1) to (41) above), the expression level of a protein (e.g., TLE1) may be tested by a method such as PCR (polymerase chain reaction), an antigen-antibody reaction, immunohistochemical staining, or DNA methylation analysis. PCR includes, for example, qPCR. The antigen-antibody reaction includes, for example, Western blotting. DNA methylation analysis includes, for example, analysis of DNA methylation upstream (e.g., in the promoter) of a gene (e.g., the TLE1 gene). For the method of testing the expression level of TLE1, a known method can be used, and for example, reference may be made to the method described in Xu et al., Mol Cell Biochem. 2018 Jan;438(1-2):85-96. When the expression level of a protein (e.g., TLE1) in a sample of a subject (e.g., a malignant tumor cell or a cell lysate thereof) is low, the degree thereof may be a degree that is significantly reduced compared to a sample from a healthy person (e.g., a normal cell or a cell lysate thereof). The degree of this low expression may be, for example, 10, 5, 3, 1, 0.1, or 0.01% or less, or 0%, or may be within a range between any two of these values. This low expression may be, for example, 10-0, 5-0, 1-0, or 0.1-0%. The degree of this low expression may be the mean value of a sample group from healthy individuals minus 1 standard deviation, or less. This value may be, for example, -1, -1.5, -2, -2.5, or -3 SD or less, or may be within a range between any two of these values. When the expression level of a protein (e.g., TLE1) in a sample of a subject is low, the degree of low expression may be the mean value of a sample group of malignant tumors of the same type as the malignant tumor that the subject has, which were obtained from patients other than the subject, minus 1 SD, or less. This value may be, for example, -1, -1.5, -2, -2.5, or -3 SD or less, or may be within a range between any two of these values. The sample group of malignant tumors obtained from patients other than the subject may be, for example, a sample group in which a high expression level of a protein (e.g., TLE1) has been confirmed in advance, or a sample group in which low sensitivity to BCV has been confirmed in advance. For example, when the malignant tumor that the subject has is a lymphoma, the malignant tumor of the same type as the malignant tumor that the subject has is a lymphoma. When the expression level of a protein (e.g., TLE1) in a sample of a subject is low, the degree of low expression may be comparable to that of a sample in which a low expression level of the protein (e.g., TLE1) has been confirmed in advance, or a sample in which high sensitivity to BCV has been confirmed in advance. Such a sample may be, for example, a cell line of KAI-3, NK-S1, NK-92, KHYG-1, FE-PD, Z4739, SUP-M2, SUDHL-1, DEL, L-82, SMZ-1, SR-786, K-299, HH, or KI-JK. This comparable degree may be, for example, a difference in expression level of ±30, 20, 10, 5, 3, 2, 1, or 0.1% or less, or 0%, or may be within a range between any two of these values. This value may be, for example, ±10-0, 5-0, 1-0, or 0.1-0%. When the expression level of a protein (e.g., TLE1) in a sample of a subject is low, the degree of low expression may be significantly lower compared to a sample with a high expression level of the protein, a sample in which a high expression level of the protein has been confirmed in advance, a sample in which the expression level of the protein is not low, or a sample in which low sensitivity to BCV has been confirmed in advance. Such a sample may be, for example, a cell line of SNK-1, SNK-6, YT, HANK-1, or SNT-8. This significantly lower degree may be, for example, 10, 5, 3, 1, 0.1, or 0.01% or less, or 0%, or may be within a range between any two of these values. This value may be, for example, 10-0, 5-0, 1-0, or 0.1-0%. The expression level may be evaluated, for example, by the amount of mRNA or the amount of protein. Considering the effect on the function of the protein, the expression level is preferably evaluated by the amount of protein. The evaluation of the expression level may include, for example, a step of comparing the expression level of the subject with a predetermined threshold, or a step of evaluating the expression level as low when the expression level of the subject is lower than the threshold. The evaluation of the expression level may include a step of evaluating the expression level as low when the expression level of the subject is significantly reduced compared to a control value. In embodiments of the present invention (e.g., (1) to (41) above or this paragraph), low TLE1 expression may be TLE1-negative, and high TLE1 expression may be TLE1-positive or TLE1 overexpression. Therefore, the above embodiments regarding low or high expression of TLE1 can be applied to embodiments of TLE1-negative or TLE1-positive, respectively. Low expression of TLE1 may be detected, for example, in a sample from a subject having an NK/T-cell lymphoma or a T-cell lymphoma. The testing of the protein expression level may be performed, for example, in vitro, ex vivo, or in vivo.

In embodiments of the present invention (e.g., (1) to (41) above), when the expression level of a protein (e.g., TLE1, ZNF441 or CERS6) in a sample of a subject (e.g., a malignant tumor cell or a cell lysate thereof) is high, the degree thereof may be a degree that is significantly increased compared to a sample from a healthy person (e.g., a normal cell or a cell lysate thereof). The degree of this high expression may be, for example, 1.3, 1.5, 2, 3, 4, 5, 10, 20, 30, 40, 50, 100, or 500 times or more, or may be within a range between any two of these values. This high expression may be, for example, 1.3-500, 2-500, 5-500, or 50-500 times. The degree of this high expression may be the mean value of a sample group from healthy individuals plus 1 standard deviation, or more. This value may be, for example, +1, +1.5, +2, +2.5, or +3 SD or more, or may be within a range between any two of these values. When the expression level of a protein (e.g., TLE1, ZNF441 or CERS6) in a sample of a subject is high, the degree of high expression may be the mean value of a sample group of malignant tumors of the same type as the malignant tumor that the subject has, which were obtained from patients other than the subject, plus 1 SD, or more. This value may be, for example, +1, +1.5, +2, +2.5, or +3 SD or more, or may be within a range between any two of these values. The sample group of malignant tumors obtained from patients other than the subject may be, for example, a sample group in which a low expression level of a protein (e.g., TLE1, ZNF441 or CERS6) has been confirmed in advance, or a sample group in which low sensitivity to BCV has been confirmed in advance. For example, when the malignant tumor that the subject has is a lymphoma, the malignant tumor of the same type as the malignant tumor that the subject has is a lymphoma. When the expression level of a protein (e.g., TLE1, ZNF441 or CERS6) in a sample of a subject is high, the degree of high expression may be comparable to that of a sample in which a high expression level of the protein (e.g., TLE1, ZNF441 or CERS6) has been confirmed in advance, or a sample in which high sensitivity to BCV has been confirmed in advance. Such a sample may be, for example, a cell line of SUDHL-5, DB, BJAB, OCI-LY19, L-428, OCI-LY18, OCI-LY3, WSU-NHL, or RL. This comparable degree may be, for example, a difference in expression level of ±30, 20, 10, 5, 3, 2, 1, or 0.1% or less, or 0%, or may be within a range between any two of these values. This value may be, for example, ±10-0, 5-0, 1-0, or 0.1-0%. When the expression level of a protein (e.g., TLE1, ZNF441 or CERS6) in a sample of a subject is high, the degree of high expression may be significantly higher compared to a sample with a low expression level of the protein, a sample in which a low expression level of the protein has been confirmed in advance, a sample in which the expression level of the protein is not high, or a sample in which low sensitivity to BCV has been confirmed in advance. Such a sample may be, for example, a cell line of SC-1 or K231. This significantly higher degree may be, for example, 1.3, 1.5, 2, 3, 4, 5, 10, 20, 30, 40, 50, 100, or 500 times or more, or may be within a range between any two of these values. This value may be, for example, 1.3-500, 2-500, 5-500, or 50-500 times. The expression level may be evaluated, for example, by the amount of mRNA or the amount of protein. Considering the effect on the function of the protein, the expression level is preferably evaluated by the amount of protein. The evaluation of the expression level may include, for example, a step of comparing the expression level of the subject with a predetermined threshold, or a step of evaluating the expression level as high when the expression level of the subject is higher than the threshold. The evaluation of the expression level may include a step of evaluating the expression level as high when the expression level of the subject is significantly increased compared to a control value. In embodiments of the present invention (e.g., (1) to (41) above or this paragraph), high expression of TLE1, ZNF441 or CERS6 may be TLE1, ZNF441 or CERS6 positivity or overexpression. Therefore, the above embodiments regarding high or low expression of TLE1, ZNF441 or CERS6 can be applied to embodiments of TLE1, ZNF441 or CERS6 positivity or negativity, respectively. High expression of TLE1 or CERS6 may be detected, for example, in a sample from a subject having a B-cell lymphoma. High expression of ZNF441 may be detected, for example, in a sample from a subject having an NK/T-cell lymphoma. The testing of the protein expression level may be performed, for example, in vitro, ex vivo, or in vivo.

In embodiments of the present invention (e.g., (1) to (41) above), the malignant tumor may include an MYC-positive, MYC-negative, EBV-positive, or EBV-negative malignant tumor. As described above, the property of being MYC-positive or negative may be used for predicting a prognosis. Positivity includes that gene expression is positive. Positivity may include that the gene expression in a sample of a subject is increased compared to the gene expression in a sample of a healthy person or a person previously confirmed to be negative. The detection of MYC or EBV positivity/negativity (or expression level) in a subject or a subject's malignant tumor may be performed, for example, by collecting a sample from the subject (e.g., a malignant tumor cell, plasma, whole blood, or tissue) and measuring the gene expression of MYC (or a gene whose expression is enhanced in association with MYC) or EBV in the sample. Gene expression can be measured, for example, by qPCR, a DNA chip, or immunostaining. In this case, a sample collected from a subject may be compared with a sample from a healthy person (e.g., a normal cell) or a sample previously confirmed to be negative, and when the gene expression of MYC or the expression of a gene whose expression is enhanced in association with MYC is significantly enhanced in the sample collected from the subject, the subject may be determined to be MYC-positive. In addition, a sample collected from a subject may be compared with a sample from a healthy person (e.g., a normal cell) or a sample previously confirmed to be negative, and when the gene expression of EBV is significantly enhanced in the sample collected from the subject, the subject may be determined to be EBV-positive. The gene expression of EBV may be examined using the expression of an EBV-derived transcript (e.g., EBER or EBNA1, etc.) as an indicator. Furthermore, the detection of MYC or EBV positivity/negativity in a subject or a subject's lymphoma may be performed, for example, by collecting a lymph node from the subject and performing immunostaining for MYC or EBV. In this case, the sample collected from the subject may be compared with a sample collected from a healthy person or a person previously confirmed to be negative, and when the staining intensity for MYC or EBV is significantly enhanced in the sample collected from the subject, the subject may be determined to be MYC- or EBV-positive. The enhancement of the gene expression or the staining intensity as described above includes, for example, an increase by a factor of 1.5, 2, 3, 4, 5, 10, 20, or 50 or more, or within a range between any two of these values, relative to a comparison subject. This factor may be, for example, 1.5-50, 1.5-10, 1.5-5, 2-50, 3-50, or 5-50 times. Furthermore, in the detection of MYC or EBV positivity/negativity in a subject or a subject's lymphoma, the subject may be determined to be MYC- or EBV-positive, for example, when the percentage of cells immunostained for MYC or EBV in a sample is a certain level or more. This percentage may be, for example, 10, 20, 30, 40, 50, 60, 70, 80, or 90% or more, or 100%, or may be within a range between any two of these values. This percentage may be, for example, 10-100, 10-70, 10-50, 20-100, 40-100, or 60-100%. Furthermore, the detection of MYC positivity/negativity in a subject or a subject's lymphoma can be performed, for example, by detecting MYC translocation via fluorescence in situ hybridization (FISH) (see, e.g., Salam et al., J Cancer 2020; 11(1):190-198 or Epperla et al., Cancer. 2017 Nov 15;123(22):4411-4418). Furthermore, the detection of EBV positivity/negativity in a subject or a subject's lymphoma can be performed, for example, by quantifying EBV DNA in plasma or whole blood using a commercially available DNA quantification kit (e.g., AccuGene m-EBV (Abbott Japan LLC)) or by detecting IgM antibodies against EBV capsid antigens in serum using a commercially available antibody detection kit (e.g., BioPlex EBV IgM Kit (Bio-Rad Laboratories, Inc.)). The detection of MYC positivity/negativity may be performed, for example, in vitro, ex vivo, or in vivo.

In embodiments of the present invention (e.g., (1) to (41) above), MYC includes a protein known as a transcriptional regulator. For example, as of the filing of the present application, the Primary accession number for MYC in UniProt is P01106. EBV is an abbreviation for Epstein-Barr virus. EBV is known as a virus having double-stranded DNA (see, e.g., Rivailler et al., J Virol. 2002 Dec; 76(23): 12055-12068 or Correia et al., J Virol. 2018 Nov 15; 92(22): e01132-18). By targeting an MYC-positive or EBV-positive lymphoma, a high therapeutic effect by BCV, a pharmaceutically acceptable salt thereof, or a solvate thereof is obtained.

In the methods of embodiments of the present invention (e.g., (1) to (29) above), the step of identifying a subject may include a step of identifying the subject as a subject to be treated. The step of identifying as a subject to be treated may include a step of identifying the subject as a subject for administration of a drug. The step of identifying a subject may include a step of selecting the subject or a step of identifying the subject.

In the methods of embodiments of the present invention (e.g., (1) to (29) above), the step of identifying a subject having a malignant tumor may be performed using a malignant tumor biomarker in a bodily fluid (e.g., blood) of the test subject as an indicator, or by diagnostic imaging (CT, etc.) or histological findings. This step of identifying may be performed using, as an indicator, the fact that the amount of the biomarker in the test subject is increased compared to the amount of the malignant tumor biomarker in a comparison subject (e.g., a healthy person).

In embodiments of the present invention (e.g., (1) to (29) above), the method may include an assisting method. The assistance may include assistance in the consideration or determination of a subject to be treated, a treatment method, or a diagnostic method. The method may be performed, for example, in vitro, ex vivo, or in vivo, depending on the purpose.

In embodiments of the present invention (e.g., (1) to (41) above), in the prediction of BCV sensitivity, a subject may be predicted to be sensitive to BCV if the expression level of TLE1 in the subject or a sample of the subject is low. In the prediction of BCV sensitivity, a subject may be predicted not to be sensitive to BCV if the expression level of TLE1 in the subject or a sample of the subject is high. In this case, the disease that the subject has may be a malignant tumor with low TLE1 expression and high sensitivity to BCV. In embodiments of the present invention (e.g., (1) to (41) above), in the prediction of BCV sensitivity, a subject may be predicted to be sensitive to BCV if the expression level of TLE1 in the subject or a sample of the subject is high. In the prediction of BCV sensitivity, a subject may be predicted not to be sensitive to BCV if the expression level of TLE1 in the subject or a sample of the subject is low. In this case, the disease that the subject has may be a malignant tumor with high TLE1 expression and high sensitivity to BCV.

In embodiments of the present invention (e.g., (1) to (41) above), in the determination of a subject with a malignant tumor to be administered BCV, a subject may be determined to be a subject to be administered BCV if the expression level of TLE1 in the subject or a sample of the subject is low. In the determination of a subject with a malignant tumor to be administered BCV, a subject may be determined to be a subject to be administered BCV at a high dose if the expression level of TLE1 in the subject or a sample of the subject is high. In this case, the malignant tumor may be a malignant tumor with low TLE1 expression and high sensitivity to BCV. In embodiments of the present invention (e.g., (1) to (41) above), in the determination of a subject with a malignant tumor to be administered BCV, a subject may be determined to be a subject to be administered BCV if the expression level of TLE1 in the subject or a sample of the subject is high. In the determination of a subject with a malignant tumor to be administered BCV, a subject may be determined to be a subject to be administered BCV at a high dose if the expression level of TLE1 in the subject or a sample of the subject is low. In this case, the malignant tumor may be a malignant tumor with high TLE1 expression and high sensitivity to BCV.

In embodiments of the present invention (e.g., (1) to (41) above), in the diagnosis of whether a subject is a subject having a malignant tumor with high sensitivity to BCV, a subject may be diagnosed as being a subject having a malignant tumor with high sensitivity to BCV if the expression level of TLE1 in the subject or a sample of the subject is low. In the diagnosis of whether a subject is a subject having a malignant tumor with high sensitivity to BCV, a subject may be diagnosed as not being a subject having a malignant tumor with high sensitivity to BCV if the expression level of TLE1 in the subject or a sample of the subject is high. In this case, the malignant tumor may be a malignant tumor with low TLE1 expression and high sensitivity to BCV. In embodiments of the present invention (e.g., (1) to (41) above), in the diagnosis of whether a subject is a subject having a malignant tumor with high sensitivity to BCV, a subject may be diagnosed as being a subject having a malignant tumor with high sensitivity to BCV if the expression level of TLE1 in the subject or a sample of the subject is high. In the diagnosis of whether a subject is a subject having a malignant tumor with high sensitivity to BCV, a subject may be diagnosed as not being a subject having a malignant tumor with high sensitivity to BCV if the expression level of TLE1 in the subject or a sample of the subject is low. In this case, the malignant tumor may be a malignant tumor with high TLE1 expression and high sensitivity to BCV.

In embodiments of the present invention (e.g., (1) to (41) above), testing includes, for example, detection or measurement. In embodiments of the present invention (e.g., (1) to (41) above), a test reagent may be, for example, a nucleic acid (e.g., a DNA strand or an RNA strand), a peptide (e.g., an oligopeptide or a polypeptide (e.g., a protein)), or a composition containing them. The nucleic acid may be, for example, a primer or a probe. The polypeptide may be, for example, an antibody. The nucleic acid may be, for example, a nucleic acid capable of binding to an upstream or downstream region of a DNA encoding a protein (e.g., TLE1) (e.g., the TLE1 gene), or a nucleic acid capable of amplifying the entire length or a part of a DNA encoding a protein (e.g., TLE1). The peptide or antibody may be, for example, a peptide or antibody capable of binding to TLE1. An oligopeptide may refer to a peptide having 1 to 20 amino acids. A polypeptide may refer to a peptide having 21 or more amino acids. The antibody may be, for example, a human, mouse, rabbit, rat, hamster, or guinea pig antibody. The antibody may be a chimeric antibody of any two or more of the above organisms. The antibody may be a monoclonal antibody. The test reagent may be implemented by replacing it with a detection means. In embodiments of the present invention (e.g., (1) to (41) above), the step of testing may be performed, for example, by PCR, an antigen-antibody reaction, immunohistochemical staining, or methylation analysis of the promoter region DNA of a gene (e.g., the TLE1 gene). The testing may be performed, for example, using the amount of a nucleic acid (e.g., a gene or mRNA), the amount of a protein, DNA methylation, or gene translocation as an indicator. The DNA methylation analysis may be performed using a means for detecting methylation or unmethylation of the promoter region of a gene (e.g., a reagent used for methylation-specific PCR, unmethylation-specific PCR, or pyrosequencing). The DNA methylation analysis may be performed using a primer (e.g., a primer capable of amplifying at least a part of the promoter region (e.g., a primer that can bind to a site upstream or downstream of the promoter region)) or an antibody. The primer may be a methylation-specific or unmethylation-specific primer. The antibody may be an antibody specific to the promoter region or a partial region thereof of a gene in a methylated or unmethylated state. The above-described test reagents may include those used in these detection steps. In embodiments of the present invention (e.g., (1) to (41) above), the step of testing the expression level may be replaced with, for example, a step of testing the expression or a step of testing for the presence of positivity/negativity.

In embodiments of the present invention (e.g., (1) to (41) above), when using the expression level of TLE1 as an indicator, the indicator may be low expression of TLE1. For example, when identifying a patient with a malignant tumor to be treated with BCV, the patient may be identified using low expression of TLE1 as an indicator. The expression level may be tested qualitatively or quantitatively. In this case, the malignant tumor may be a malignant tumor with low TLE1 expression and high sensitivity to BCV. In embodiments of the present invention (e.g., (1) to (41) above), when using the expression level of TLE1 as an indicator, the indicator may be high expression of TLE1. For example, when identifying a patient with a malignant tumor to be treated with BCV, the patient may be identified using high expression of TLE1 as an indicator. The expression level may be tested qualitatively or quantitatively. In this case, the malignant tumor may be a malignant tumor with high TLE1 expression and high sensitivity to BCV.

In embodiments of the present invention (e.g., (1) to (41) above), the sample may include, for example, a biological sample. The sample may include, for example, a cell (e.g., a malignant tumor cell), a cell lysate, a blood specimen (e.g., plasma, serum, or whole blood), or a tissue. The sample may include, for example, a specimen. Instead of the sample, malignant tumor cells of a subject with a malignant tumor may be used.

In embodiments of the present invention (e.g., (1) to (41) above), a more appropriate BCV treatment may be a treatment that provides a higher therapeutic effect compared to a BCV treatment that does not use the expression level of TLE1 as an indicator or that does not consider BCV sensitivity. A more appropriate BCV treatment may be a treatment administered to a population in which the treatment is more effective, a treatment administered to a subject for whom a therapeutic effect is more likely to appear, or a treatment in which a more appropriate treatment regimen is implemented. "A therapeutic effect appearing" may include that the tumor is significantly reduced compared to before the administration of the active ingredient or upon administration of a non-active ingredient (e.g., a placebo).

In embodiments of the present invention (e.g., (1) to (41) above), treatment includes being able to exert an action that produces a beneficial result for a patient with respect to the patient's disease (e.g., a malignant tumor (e.g., a lymphoma)) or one or more symptoms associated with the disease (e.g., amelioration, mitigation, alleviation, cure, remission, or suppression (e.g., suppression of onset (e.g., prevention), suppression of progression, or suppression of recurrence)). The treatment may be performed by administering a therapeutically effective amount of BCV, a pharmaceutically acceptable salt thereof, or a solvate thereof to the subject. In embodiments of the present invention (e.g., (1) to (41) above), the pharmaceutical composition may be produced by any method known in the technical field of pharmaceutics, wherein, for example, an active ingredient and one or more pharmaceutically acceptable carriers are mixed. Furthermore, the pharmaceutical composition is not limited in its form of use as long as it is used for treatment, and may be the active ingredient alone or a mixture of the active ingredient and any optional ingredient(s). The form of the above-described carrier is not particularly limited and may be, for example, a solid or a liquid (e.g., a buffer). The content of the above-described carrier may be, for example, a pharmaceutically effective amount. The effective amount may be, for example, an amount sufficient for the pharmaceutical stability or delivery of the active ingredient. For example, a buffer is effective in stabilizing the active ingredient in a vial. The pharmaceutical composition may also contain a stabilizer (e.g., mannitol), a buffering agent (e.g., arginine), or a pH adjusting agent (e.g., NaOH). The dosage, dosing interval, administration method, and administration route are not particularly limited and may be selected as appropriate based on factors such as the patient's age, body weight, symptoms, or the target organ. The pharmaceutical composition preferably contains an active ingredient in a therapeutically effective amount or an amount effective to exert the desired action. In one embodiment of the present invention, the therapeutically effective amount includes an amount necessary for achieving a clinically observable improvement of symptoms in a patient. In one embodiment of the present invention, "pharmaceutically acceptable" includes a state suitable for use that is within the scope of sound medical judgment and is commensurate with a reasonable benefit/risk ratio. Ingredients other than BCV in the pharmaceutical composition are not particularly limited as long as they do not impair the effects of the present invention and may be selected as appropriate depending on the purpose. The therapeutic effect on a malignant tumor may be considered present when the patient's malignant tumor cells are significantly reduced after administration of a therapeutic agent.

In embodiments of the present invention (e.g., (1) to (41) above), the subject (including a patient) may be, for example, a subject diagnosed as having a malignant tumor, a subject suspected of having a malignant tumor, or a subject in need of treatment for a malignant tumor. The subject includes a population of subjects. The subject may be, for example, a subject who is predicted to have high sensitivity to BCV, a pharmaceutically acceptable salt thereof, or a solvate thereof. The subject may be, for example, a subject having a malignant tumor with a low expression level of TLE1 (a subject with a malignant tumor with low TLE1 expression). The subject may be, for example, a subject having a malignant tumor with a high expression level of TLE1 (a subject with a malignant tumor with high TLE1 expression). A subject with a malignant tumor includes a subject having a malignant tumor. The subject may be, for example, a subject in whom a biomarker for a malignant tumor is increased compared to a comparison subject (e.g., a healthy person), or a subject in whom a malignant tumor has been observed by diagnostic imaging or histological findings. The subject may be, for example, a subject who has experienced a malignant tumor. The subject may be, for example, a subject diagnosed as having a malignant tumor with low TLE1 expression. The subject may be, for example, a subject diagnosed as having a malignant tumor with high TLE1 expression. The subject includes a human or a non-human mammal (e.g., one or more of a mouse, guinea pig, hamster, rat, rabbit, pig, sheep, goat, cow, horse, cat, dog, marmoset, monkey, or chimpanzee). The subject includes a human adult patient.

In embodiments of the present invention (e.g., (1) to (41) above), the administration route of BCV, a pharmaceutically acceptable salt thereof, or a solvate thereof (or a pharmaceutical composition containing them) to a subject (e.g., a human subject) is preferably one that is effective for treatment, and may be, for example, intravenous, oral, subcutaneous, intramuscular, or intraperitoneal. The dosage form is preferably one that is effective for treatment and may be, for example, a solid formulation (e.g., a tablet), a liquid formulation (e.g., an oral solution), or an injectable formulation (e.g., an intravenous injection).

In embodiments of the present invention (e.g., (1) to (41) above), the dosage, dosing interval, and administration method of BCV, a pharmaceutically acceptable salt thereof, or a solvate thereof (or a pharmaceutical composition containing them) to a subject (e.g., a human subject) may be selected as appropriate based on factors such as the patient's age, body weight, symptoms, or the target organ. The dosage may be, for example, 0.01 to 200 mg/kg body weight per administration. The dosing interval may be, for example, one or two administrations every 1 to 28 days or every 1 to 4 weeks. More specifically, it includes intravenous injection at 20 to 80 mg/day. It may also include intravenous injection at 20 to 80 mg/body/day twice a week. In combination treatment with BCV and a chemotherapeutic agent (e.g., gemcitabine, etoposide, or an immune checkpoint inhibitor), the dosage of BCV may include intravenous injection at 10 to 40 mg or 10 to 20 mg/body/day. It may also include intravenous injection at 10 to 40 mg or 10 to 20 mg/body/day twice a week. The range of 10 to 80 mg/body/day indicated herein may be, for example, 10, 20, 30, 40, 50, 60, 70, or 80 mg/body/day, or may be within a range between any two of these values. In embodiments of the present invention (e.g., (1) to (41) above), a low dose of BCV, a pharmaceutically acceptable salt thereof, or a solvate thereof may include, for example, 5-80, 10-40, or 10-60 mg/body. A high dose may include, for example, 90-200, 100-180, or 120-160 mg/body/day.

In embodiments of the present invention (e.g., (1) to (41) above), the salt is not particularly limited and includes, for example, an inorganic salt or an organic salt (see, e.g., Bharate et al., Drug Discov Today. 2021 Feb;26(2):384-398. or Berge et al., J Pharm Sci. 1977 Jan;66(1):1-19.). The salt includes, for example, a metal salt, an ammonium salt, a salt with an organic base, a salt with an inorganic acid, a salt with an organic acid, a salt with a basic or acidic amino acid, and the like. Metal salts include, for example, alkali metal salts (sodium salts, potassium salts, etc.), alkaline earth metal salts (calcium salts, magnesium salts, barium salts, etc.), aluminum salts, and the like. Salts with organic bases include, for example, salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, and the like. Salts with inorganic acids include, for example, salts with hydrochloric acid, hydrobromic acid, nitric acid, sulfuric acid, phosphoric acid, and the like. Salts with organic acids include, for example, salts with formic acid, acetic acid, trifluoroacetic acid, phthalic acid, fumaric acid, mesylic acid, tosylic acid, oxalic acid, tartaric acid, maleic acid, citric acid, succinic acid, malic acid, methanesulfonic acid, benzenesulfonic acid, p-toluenesulfonic acid, and the like. Salts with basic amino acids include, for example, salts with arginine, lysine, ornithine, and the like. Salts with acidic amino acids include, for example, salts with aspartic acid, glutamic acid, and the like. The salt includes a pharmaceutically acceptable salt. In one embodiment of the present invention, "pharmaceutically acceptable" includes a form that has a reasonable benefit for pharmaceutical use. In one embodiment of the present invention, one form of a compound or a salt thereof includes a form of a solvate thereof. In embodiments of the present invention (e.g., (1) to (41) above), a solvate includes a form of a compound formed by a solute and a solvent (see, e.g., Healy et al., Adv Drug Deliv Rev. 2017 Aug 1;117:25-46.). The solvate includes, but is not particularly limited to, for example, a hydrate (e.g., a monohydrate, dihydrate, trihydrate, etc.) or a solvate with an organic solvent (e.g., a solvate with an alcohol (methanol, ethanol, propanol, etc.), acetone, dimethylformamide, or ethyl acetate, etc.). The solvent includes a solvent that can substantially maintain the physiological activity of a solute after forming a solvate. The solvate includes a pharmaceutically acceptable solvate.

In one embodiment of the present invention, "significantly" may refer to, for example, unless otherwise specified, a state in which a statistical significance may be evaluated using Student's t-test (one-tailed or two-tailed), and the p-value is <0.05 or <0.01. Alternatively, it may refer to a state in which a substantial difference exists.

All publications cited herein are incorporated by reference in their entirety. As used herein, the term "or" is used when "at least one or more" of the items listed in the text may be adopted. The same applies to "or". As used herein, a term in the format "at least one or more of A, B, and C" means that any of (A), (B), (C), (A and B), (A and C), (B and C), or (A, B, and C) can be adopted. As used herein, when "within a range of two values" is specified, the range includes the two values themselves. As used herein, "A to B" includes A and B and the values contained between A and B. As used herein, "a", "an", and "the" may be intended to mean that one or more elements or steps may be present, unless the context clearly indicates otherwise. As used herein, "having" includes suffering from a disease when it relates to a disease. As used herein, "the above (1) to (41)" includes a reference to any one or more of (1), (2), (3), (4), (5), (6), (7), (8), (9), (10), (11), (12), (13), (14), (15), (16), (17), (18), (19), (20), (21), (22), (23), (24), (25), (26), (27), (28), (29), (30), (31), (32), (33), (34), (35), (36), (37), (38), (39), (40), or (41).

While embodiments of the present invention have been described above, these are illustrative of forms that may be included in the present invention, the present invention is not limited to these, and various configurations other than those described above may also be adopted. In addition, the present invention may also adopt the respective configurations or features described in the above embodiments in combination or independently.

### Examples

Hereinbelow, the present invention will be described in more detail with reference to Examples. However, the present invention is not limited to them.

### Example 1: Effect of BCV on the viability of malignant tumor cells

The effect of BCV on cell viability was examined in 11 different NK/T-cell lymphoma cell lines. Cell viability was quantified using Promega CellTiter-Glo(registered trademark) 2.0 Cell Viability Assay (Promega, Madison, Wisconsin, USA) according to the manufacturer's protocol. Specifically, cells were plated in a 96-well plate at a concentration of 2×10³ cells in 100 µL of culture medium, and the drug (BCV) was added at each concentration. After each time point, Promega CellTiter-Glo (registered trademark) 2.0 Cell Viability Assay reagent was added to the wells and incubated at room temperature for 10 minutes, and subsequently, absorbance at 480 nm was measured using a Tecan M200 Infinite 96-well plate reader and iControl software 1.6 (Tecan, Männedorf, Switzerland). Cell viability was calculated as a percentage of the control absorbance. The growth-inhibitory effect was analyzed by generating a dose-response curve as a plot of the percentage of viable cells against the drug concentration, and their IC₅₀s were estimated using GraphPad Prism version 8.0.2 (GraphPad Software). All reactions were performed in triplicate.

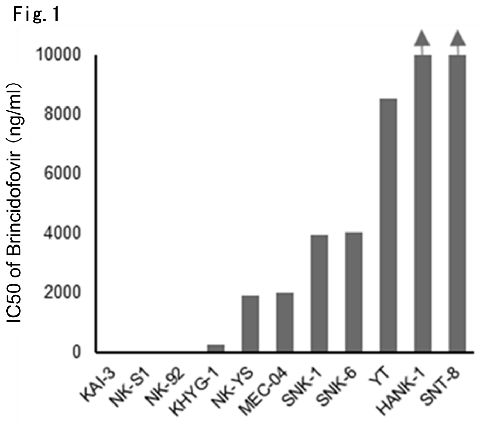

The results are shown in Fig. 1. The addition of BCV decreased the viability of NK/T-cell lymphoma cells. Sensitivity to BCV varied for each cell line.

### Example 2: In vivo study

0.5 x 10⁶ cells of NK-S1 were inoculated into 6-week-old female NSG mice, and BCV or a control solvent was administered intraperitoneally at a dosage of 40 mg/kg twice weekly. Tumor measurements were recorded twice weekly until the tumor size in the control group reached approximately 2000 mm³. Mice were euthanized according to the IACUC guidelines. The tumor sizes of the experimental group and the control group (n=8 per group) were averaged at each time point (Days 1, 5, 8, 12, and 15) and compared statistically. Throughout the experiment, signs of toxicity including diarrhea and weight loss were monitored.

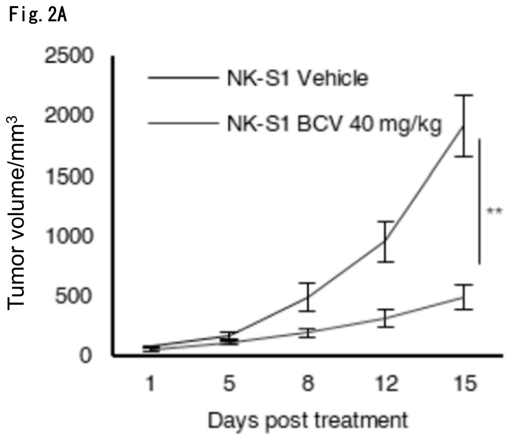

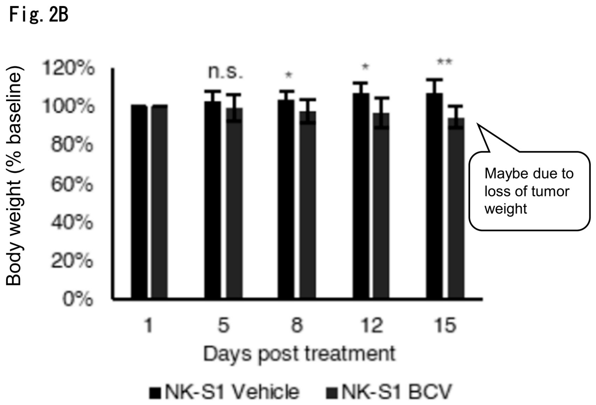

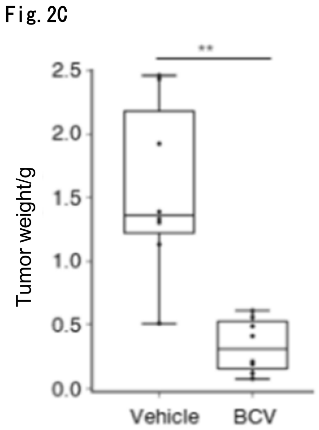

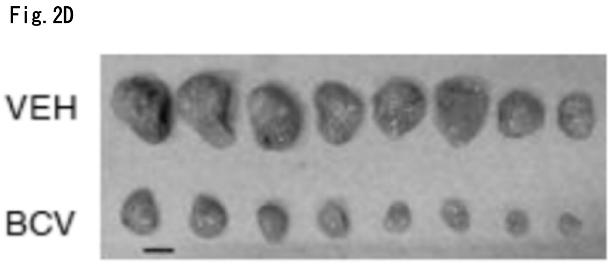

The results are shown in Figs. 2A-D. Administration of BCV significantly suppressed the enlargement of lymphoma, and the animal's health status was good with no diarrhea observed.

### Example 3: Whole transcriptome sequence analysis

Whole transcriptome sequencing was performed on the same 11 NK/T-cell lymphoma cell lines as in Example 1. Genes differentially expressed between the top four most BCV-sensitive cell lines (KAI-3, NK-S1, NK-92, KHYG-1) and the remaining seven cell lines were identified using DESeq2 (Love MI, Huber W, Anders S. Moderated estimation of fold change and dispersion for RNA-seq data with DESeq2. Genome Biol. 2014, 15(12):550) and are shown in a Volcano plot.

The results are shown in Fig. 3. TLE1 was found as the gene with the most decreased expression in the top four cell lines described above. This result indicates that TLE1 can be an indicator for predicting sensitivity to BCV. This result also indicates that when BCV is administered to a patient having an NK/T-cell lymphoma with low TLE1 expression, an improvement in the therapeutic effect or an increase in the probability of a therapeutic effect appearing can be expected, as compared to when BCV is administered to an unspecified patient.

At the same time, ZNF441 was found as a gene with enhanced expression and a strong association with BCV sensitivity, indicating that it could be an indicator for predicting sensitivity, similar to TLE1. In addition, the gene groups described in the figure may be complementary indicators for predicting sensitivity.

### Example 4: TLE1 expression level analysis

Cell lysates from each of the same 11 NK/T-cell lymphoma cell lines as in Example 1 were separated by SDS-PAGE using 4-15% Mini-PROTEAN^{™} TGX Stain-Free^{™} Protein Gels (Bio-Rad Laboratories, Hercules, CA, USA), and the separated components were transferred to a 0.2 µm PVDF membrane (Bio-Rad Laboratories, Hercules, California, USA). After blocking for 1 hour in a TBST solution (50 mM Tris/HCl, pH 7.4, 150 mM NaCl, 0.1% Tween-20) containing 5% skimmed milk powder (Bio-Rad Laboratories, Hercules, CA, USA) or 5% bovine serum albumin (Sigma-Aldrich, Darmstadt, Germany), the membrane was exposed to a primary antibody for TLE1 (Abcam) overnight at 4°C with gentle shaking. Furthermore, exposure to an HRP-conjugated anti-rabbit or anti-mouse antibody (Cytiva, Washington D.C., USA) was performed for 1 hour, and finally, chemiluminescent detection was performed using a SuperSignal Substrate Western Blotting Kit (Thermo Fisher Scientific, MA, USA) and imaged on a ChemiDoc^{™} XRS+ system (Bio-Rad Laboratories, Hercules, California, USA) using Image Lab^{™} software.

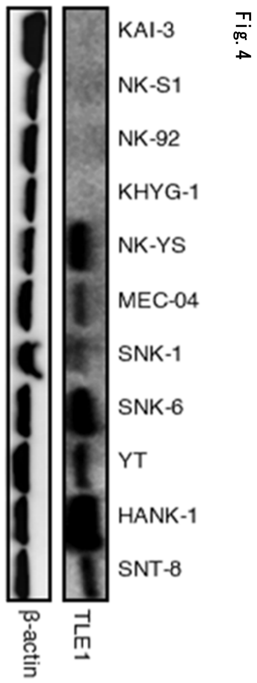

The results are shown in Fig. 4. The expression level of TLE1 in the top four most BCV-sensitive cell lines was clearly lower than in the remaining cell lines.

### Example 5: Gene mutation analysis

Somatic gene mutation information for the same 11 NK/T-cell lymphoma cell lines as in Example 1 was obtained by whole-genome sequencing followed by variant calling using an in-house bioinformatics pipeline. Whole-genome sequencing was performed on an Illumina HiSeq X platform as paired-end 150 bp reads.

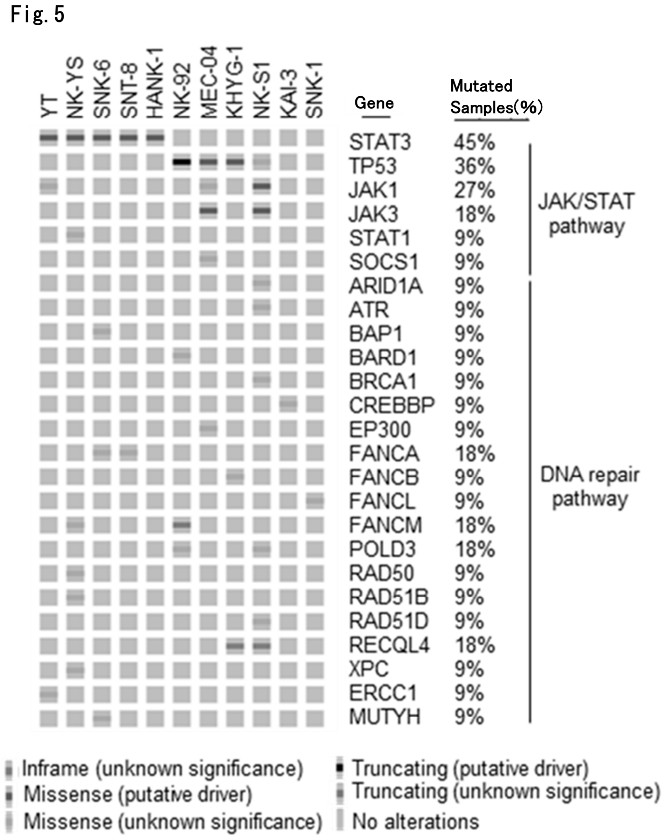

The results are shown in Fig. 5. No clear association was found between the 25 somatic gene mutations examined and BCV sensitivity.

### Example 6: Gene set expression analysis

Whole transcriptome sequencing was performed on the same 11 NK/T-cell lymphoma cell lines as in Example 1. Gene set enrichment analysis (GSEA) was performed using the Molecular Signatures Database (MSigDB) Hallmark gene set to compare the top four most BCV-sensitive cell lines. A gene set was considered significantly varied when the False Discovery Rate (FDR) q-value of the normalized enrichment score (NES) was less than 0.05.

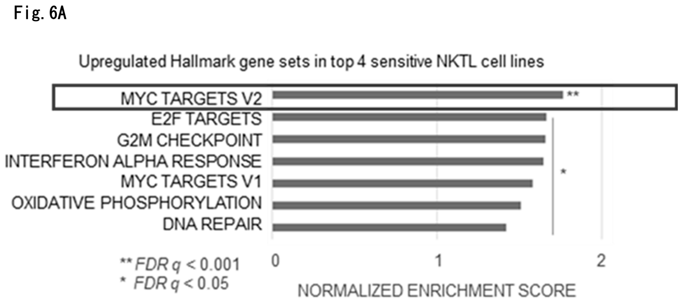

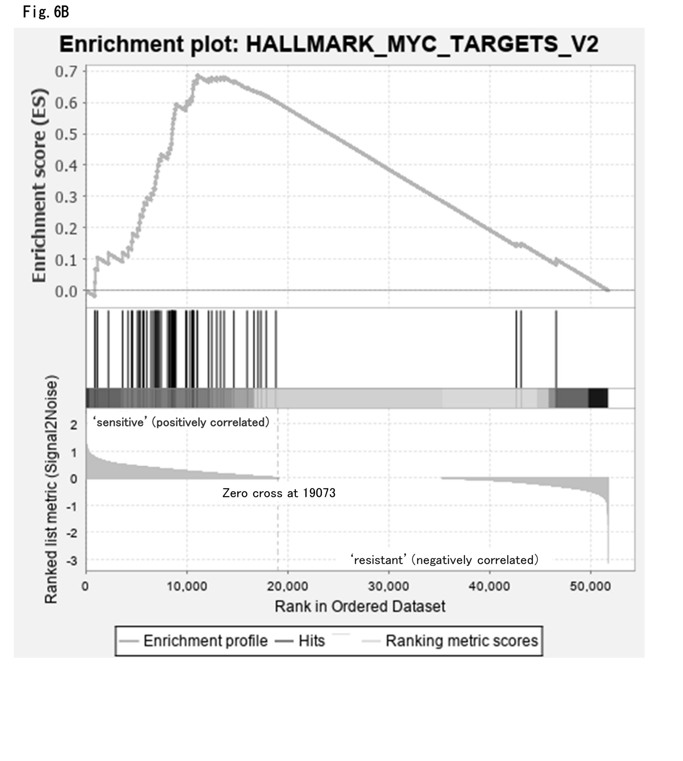

The results are shown in Figs. 6A-B. Various gene sets, including the MYC target gene group, showed a significant increase in expression in the top four cell lines.

### Example 7: Effect of TLE1 expression level on prognosis

The association between the TLE1 gene expression level obtained from whole transcriptome sequencing in an NK/T-cell lymphoma patient cohort (n=36) and progression-free survival and overall survival was examined. TLE1 gene expression exceeding the median level was defined as high TLE1 expression. To evaluate TLE1 expression as a predictor of survival, Kaplan-Meier analysis was performed, and for progression-free survival, the hazard ratio (HR) was 3.44, and the 95% confidence interval was 1.23-10.49. Statistical analysis was performed using MedCalc for Windows, version 18.2.1 (MedCalc Software).

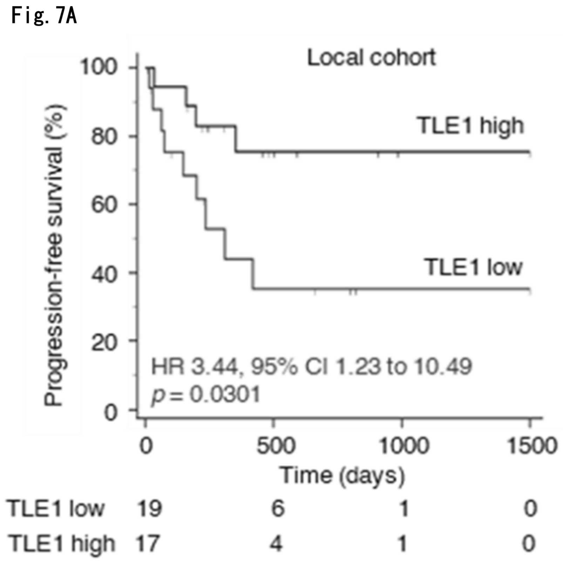

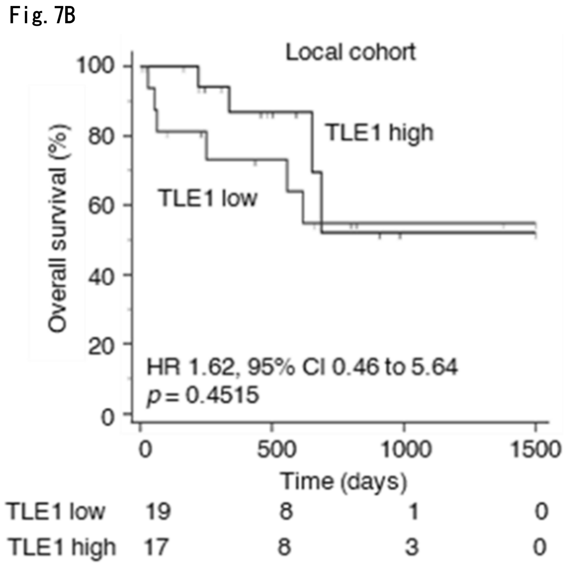

The results are shown in Figs. 7A-B. It was revealed that in NK/T-cell lymphoma patients, a low expression level of TLE1 results in a poor prognosis.

### Example 8: Gene set expression analysis

Based on the results obtained from whole transcriptome sequencing in the same NK/T lymphoma patient cohort (n=36) as in Example 7, Gene set enrichment analysis (GSEA) was performed using the Molecular Signatures Database (MSigDB) Hallmark gene set to compare low TLE1 expression patient samples and high TLE1 expression patient samples. A gene set was considered significantly varied when the False Discovery Rate (FDR) q-value of the normalized enrichment score (NES) was less than 0.05.

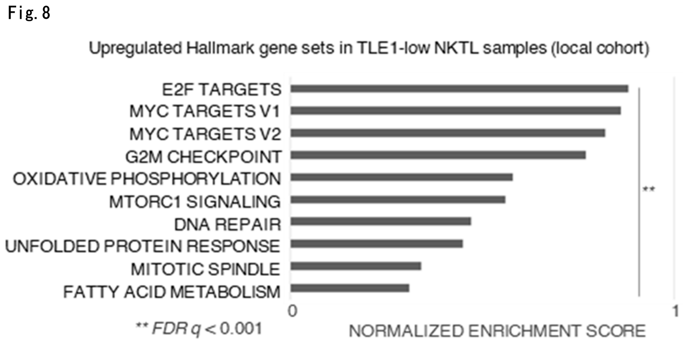

The results are shown in Fig. 8. Similar to the results obtained using NK/T-cell lymphoma cell lines, various gene sets, including the MYC target gene group, showed a significant increase in expression in patients with low TLE1 expression.

### Example 9: Effect of BCV on the viability of malignant tumor cells

The effect of BCV on the viability of 11 different T-cell lymphoma cell lines was examined. Cell viability was quantified using Promega CellTiter-Glo(registered trademark) 2.0 Cell Viability Assay (Promega, Madison, Wisconsin, USA) according to the manufacturer's protocol. Specifically, cells were plated in a 96-well plate at a concentration of 2×10³ cells in 100 µL of culture medium, and the drug was added at each concentration. After each time point, Promega CellTiter-Glo(registered trademark) 2.0 Cell Viability Assay reagent was added to the wells and incubated at room temperature for 10 minutes, and subsequently, absorbance at 480 nm was measured using a Tecan M200 Infinite 96-well plate reader and iControl software 1.6 (Tecan, Männedorf, Switzerland). Cell viability was calculated as a percentage of a control absorbance. The growth-inhibitory effect was analyzed by generating a dose-response curve as a plot of the percentage of viable cells against the drug concentration, and their IC₅₀s were estimated using GraphPad Prism version 8.0.2 (GraphPad Software). All reactions were performed in triplicate.

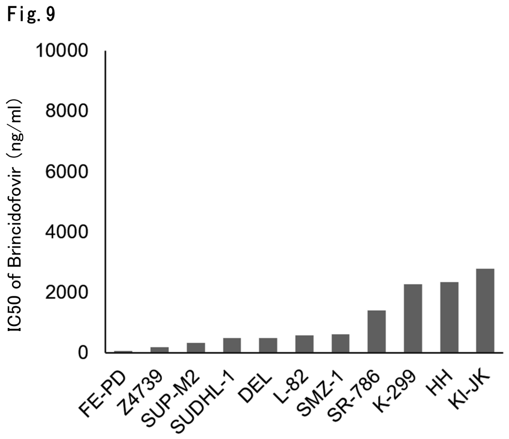

The results are shown in Fig. 9. The addition of BCV decreased the viability of T-cell lymphoma cells. Sensitivity to BCV was unexpectedly found to be high in all cell lines. This indicated that T-cell lymphoma is particularly suitable as a therapeutic target for BCV compared to other malignant tumors.

### Example 10: In vivo study

0.5 x 10⁶ cells of Z4739 were inoculated into 6-week-old female NSG mice, and BCV or a control solvent was administered intraperitoneally at a dosage of 40 mg/kg twice weekly. Tumor measurements were recorded at each time point (Days 4, 7, 11, 18, and 21) until the tumor size in the control group reached approximately 2500 mm³. Mice were euthanized according to the IACUC guidelines. The tumor sizes and weights of the experimental group and the control group (n=5 per group) were averaged on Day 21 and compared statistically. Throughout the experiment, signs of toxicity including diarrhea and weight loss were monitored.

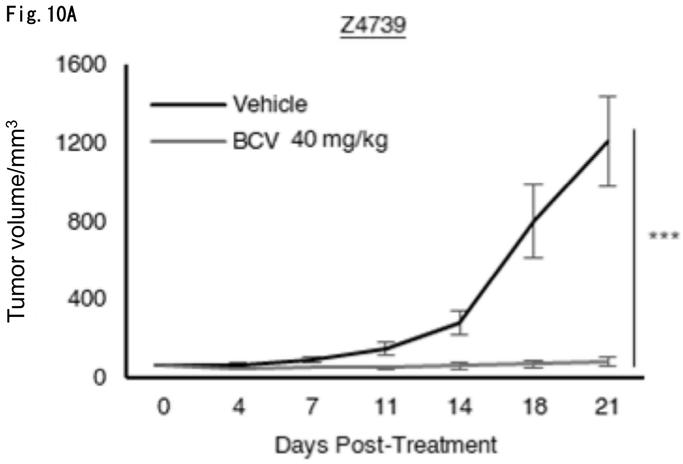

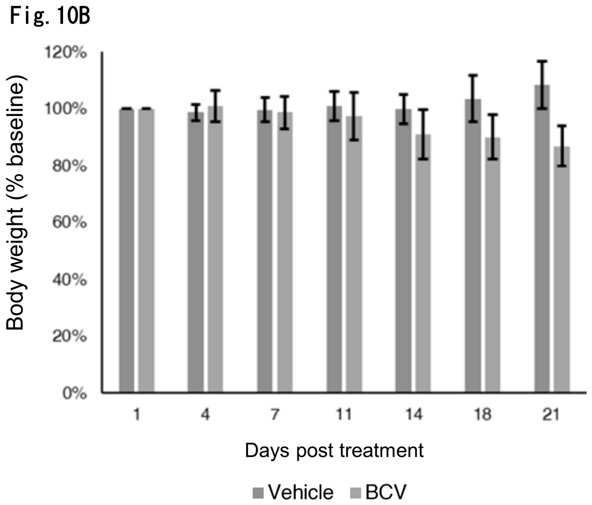

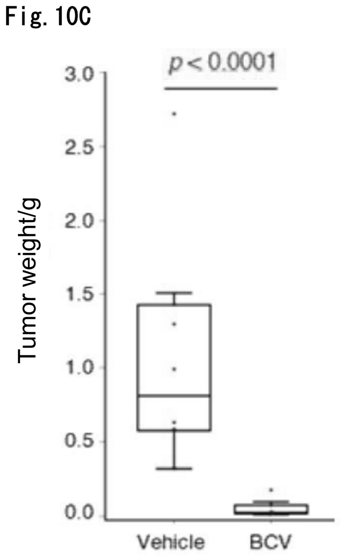

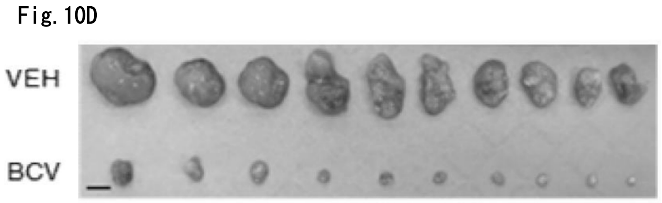

The results are shown in Figs. 10A-D. In the vehicle-administered group, a marked enlargement of the lymphoma was observed. On the other hand, in the BCV-administered group, the enlargement of the lymphoma was significantly suppressed. Furthermore, in the BCV-administered group, the animal's health status was good, and no significant weight loss or diarrhea was observed (Fig. 10B). It was a particularly unexpected result that the tumor volume in the BCV-administered group remained consistently low between Day 0 and Day 21, with almost no increase in tumor volume observed (Fig. 10A). This indicated that T-cell lymphoma is particularly suitable as a therapeutic target for BCV compared to other malignant tumors.

### Example 11: Whole transcriptome sequence analysis

Whole transcriptome sequencing was performed respectively for (i) the top four most BCV-sensitive cell lines described in Example 1 (KAI-3, NK-S1, NK-92, KHYG-1), (ii) the remaining seven cell lines (NK-YS, MEC-04, SNK-1, SNK-6, YT, HANK-1, SNT-8), and (iii) the 11 cell lines that showed high sensitivity to BCV as described in Example 9 (FE-PD, Z4739, SUP-M2, SUDHL-1, DEL, L-82, SMZ-1, SR-786, K-299, HH, KI-JK). The TLE1 gene expression was evaluated from the obtained data.

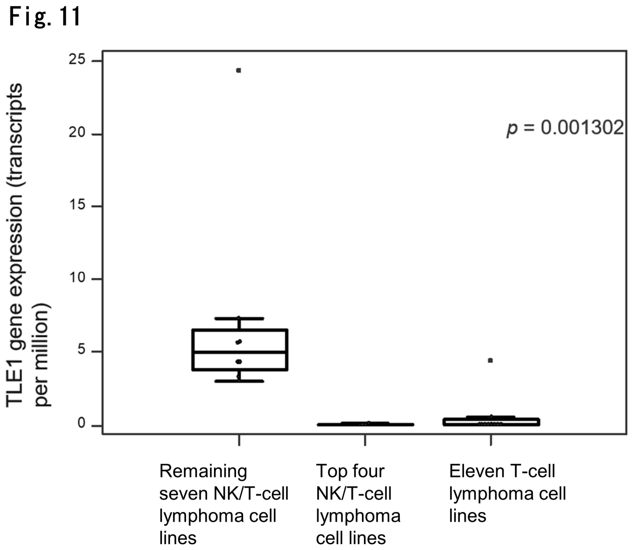

The results are shown in Fig. 11. The group of four cell lines and the group of 11 cell lines described above had significantly lower TLE1 gene expression compared to the group of seven cell lines (p=0.001302, Kruskal-Wallis test). This result indicates that TLE1 can be an indicator for predicting sensitivity to BCV. This result also indicates that when BCV is administered to a patient having an NK/T-cell lymphoma or a T-cell lymphoma with low TLE1 expression, an improvement in the therapeutic effect or an increase in the probability of a therapeutic effect appearing can be expected, as compared to when BCV is administered to an unspecified patient.

### Example 12: Effect of BCV on gene expression in malignant tumor cells

The effect of BCV on the gene expression of a T-cell lymphoma cell line was examined. Whole transcriptome sequencing of the Z4739 cell line treated with 0.1 and 1 µg/mL of BCV was performed, and Gene set enrichment analysis (GSEA) was performed using the Molecular Signatures Database (MSigDB) Hallmark gene set. A gene set was considered significantly enriched when the False Discovery Rate (FDR) q-value of the Normalized Enrichment Score (NES) was less than 0.05. Individual gene expression profiles (IFNB1, IFNY, CCL5, CXCL10, MYC) obtained from the whole transcriptome sequencing results were expressed as the number of transcripts detected per million reads.

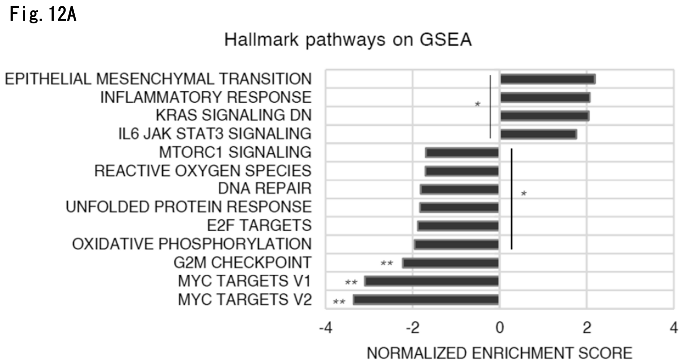

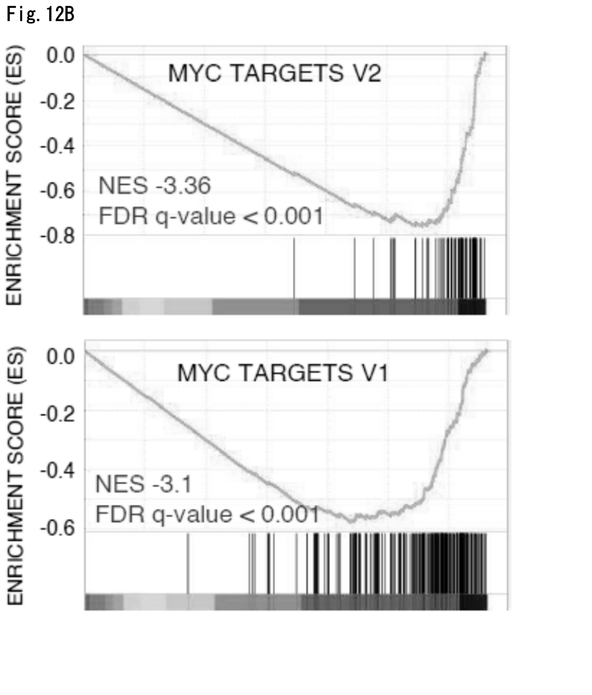

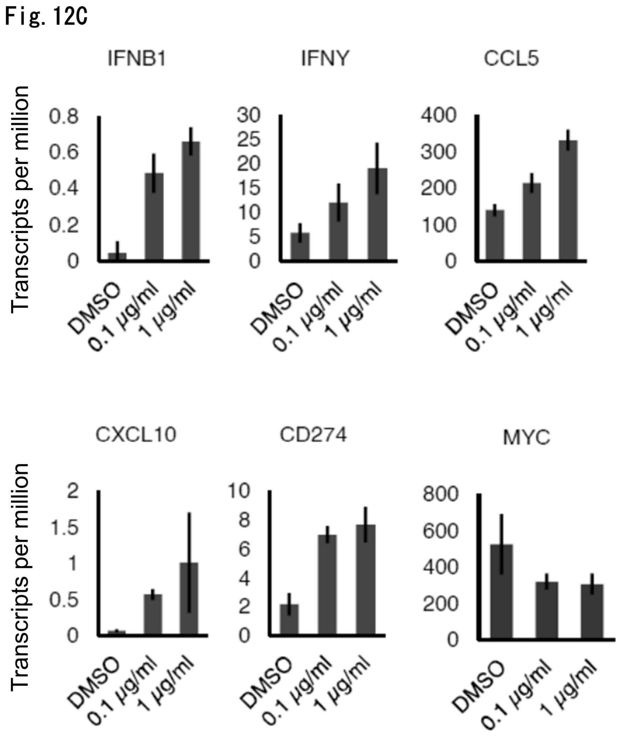

The results are shown in Figs. 12A-C. Administration of BCV decreased the expression of MYC, the expression of MYC-related gene groups, and the expression of other oncogenic genes such as those related to mTOR. On the other hand, the increased expression of immune activation-related genes such as INFγ suggests the utility of BCV for tumors in which oncogenic genes are enhanced or for tumors in which cancer immunity is suppressed.

### Example 13: Effect of BCV on gene expression in malignant tumor cells (Results of Western Blotting)

Lysates of Z4739 cells treated with 0.1 and 1 µg/mL of BCV were separated by SDS-PAGE using 4-15% Mini-PROTEAN^{™} TGX Stain-Free^{™} Protein Gels (Bio-Rad Laboratories, Hercules, CA, USA), and the separated components were transferred to a 0.2 µm PVDF membrane (Bio-Rad Laboratories, Hercules, California, USA). After blocking for 1 hour in a TBST solution (50 mM Tris/HCl, pH 7.4, 150 mM NaCl, 0.1% Tween-20) containing 5% skimmed milk powder (Bio-Rad Laboratories, Hercules, CA, USA) or 5% bovine serum albumin (Sigma-Aldrich, Darmstadt, Germany), the membrane was exposed to respective primary antibodies overnight at 4°C with gentle shaking. Furthermore, exposure to an HRP-conjugated anti-rabbit or anti-mouse antibody (Cytiva, Washington D.C., USA) was performed for 1 hour, and finally, chemiluminescent detection was performed using a SuperSignal Substrate Western Blotting Kit (Thermo Fisher Scientific, MA, USA) and imaged on a ChemiDoc^{™} XRS+ system (Bio-Rad Laboratories, Hercules, California, USA) using Image Lab^{™} software.

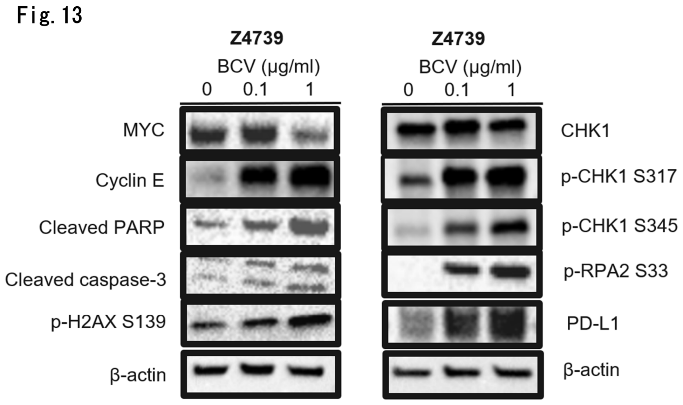

The results are shown in Fig. 13. BCV treatment led to a decrease in MYC protein and an increase in DNA damage (phospho-H2AX), as well as an increase in apoptosis indicators (cleaved PARP, cleaved caspase-3), cell cycle-related indicators (phospho-CHK1 Ser317/Ser345), and PD-L1, suggesting the presumed mechanism of the antitumor action of BCV.

### Example 14: Effect of BCV on the viability of malignant tumor cells

The effect of BCV on cell viability was examined in 19 different B-cell lymphoma cell lines and a CML cell line (K-562) as a control. Cell viability was quantified using Promega CellTiter-Glo(registered trademark) 2.0 Cell Viability Assay (Promega, Madison, Wisconsin, USA) according to the manufacturer's protocol. Specifically, cells were plated in a 96-well plate at a concentration of 2×10³ cells in 100 µL of culture medium, and the drug (BCV) was added at each concentration. After each time point, Promega CellTiter-Glo(registered trademark) 2.0 Cell Viability Assay reagent was added to the wells and incubated at room temperature for 10 minutes, and subsequently, absorbance at 480 nm was measured using a Tecan M200 Infinite 96-well plate reader and iControl software 1.6 (Tecan, Männedorf, Switzerland). Cell viability was calculated as a percentage of a control absorbance. The growth-inhibitory effect was analyzed by generating a dose-response curve as a plot of the percentage of viable cells against the drug concentration, and their IC₅₀s were estimated using GraphPad Prism version 8.0.2 (GraphPad Software). All reactions were performed in triplicate.

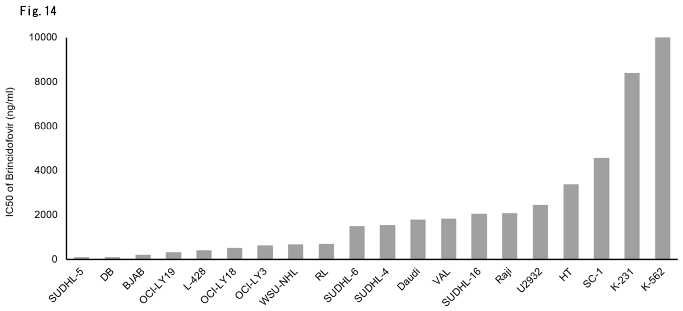

The results are shown in Fig. 14. The addition of BCV decreased the viability of B-cell lymphoma cells. Sensitivity to BCV varied for each cell line.

### Example 15: In vivo study

0.5 x 10⁶ cells of OCI-LY19 were inoculated into 6-week-old female NSG mice, and BCV or a control solvent was administered intraperitoneally at a dosage of 40 mg/kg twice weekly. Tumor measurements were recorded at each time point (Days 1, 5, 6, and 7) until the tumor size in the control group reached approximately 2500 mm³. Mice were euthanized according to the IACUC guidelines. The tumor sizes and weights of the experimental group and the control group (n=5 per group) were averaged on Day 7 and compared statistically. Throughout the experiment, signs of toxicity including diarrhea and weight loss were monitored.

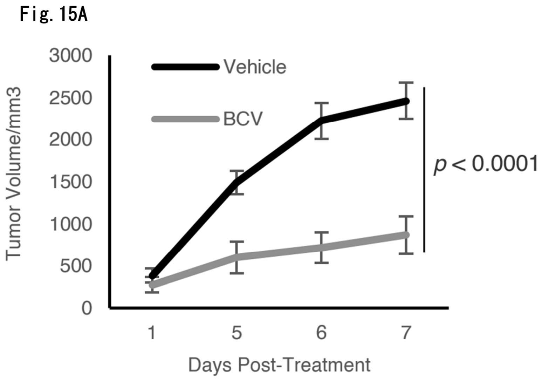

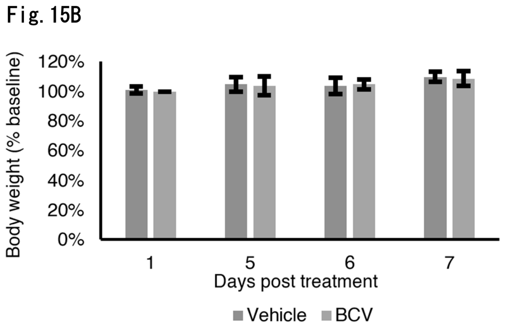

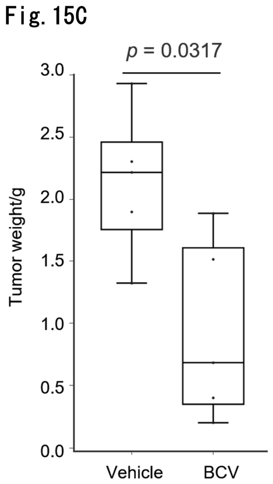

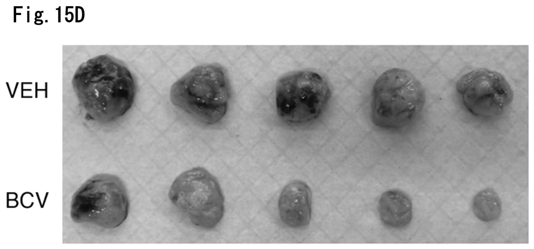

The results are shown in Figs. 15A-D. Administration of BCV significantly suppressed the enlargement of lymphoma, and the animal's health status was good with no weight loss or diarrhea observed.

### Example 16: Whole transcriptome sequence analysis

Whole transcriptome sequencing was performed on the same 19 B-cell lymphoma cell lines as in Example 9. Genes differentially expressed between the top nine most BCV-sensitive cell lines (SUDHL5, DB, BJAB, OCI-LY19, L-428, OCI-LY18, OCI-LY3, WSU-NHL, RL) and the remaining 10 cell lines were identified using DESeq2 (Love MI, Huber W, Anders S. Moderated estimation of fold change and dispersion for RNA-seq data with DESeq2. Genome Biol. 2014, 15(12):550) and are shown in a Volcano plot.

The results are shown in Fig. 16. TLE1 was found as one of the genes with increased expression in the top nine cell lines described above. This result indicates that TLE1 can be an indicator for predicting sensitivity to BCV. This result also indicates that when BCV is administered to a patient having a B-cell lymphoma with high TLE1 expression, an improvement in the therapeutic effect or an increase in the probability of a therapeutic effect appearing can be expected, as compared to when BCV is administered to an unspecified patient.

At the same time, CERS6 was found as the gene whose enhanced expression was most correlated with sensitivity, indicating that it could be an indicator for predicting sensitivity, similar to TLE1. In addition, the gene groups described in the figure may be complementary indicators for predicting sensitivity.

### Example 17: Patient cohort analysis

Regarding previously reported gene analysis data for a B-cell lymphoma patient cohort, the expression level of TLE1 was analyzed. Based on gene expression data from two different previously reported DLBCL patient cohorts, a) GSE11318 and b) GSE10846, the association between TLE1 gene expression level and overall survival was examined. TLE1 gene expression exceeding the median level was defined as high TLE1 expression. To evaluate TLE1 expression as a predictor of survival, Kaplan-Meier analysis was performed, and in a) GSE11318, the hazard ratio (HR) was 2.77, and the 95% confidence interval was 1.78-4.30, and in b) GSE10846, the hazard ratio (HR) was 1.94, and the 95% confidence interval was 1.35-2.80. Statistical analysis was performed using MedCalc for Windows, version 18.2.1 (MedCalc Software).

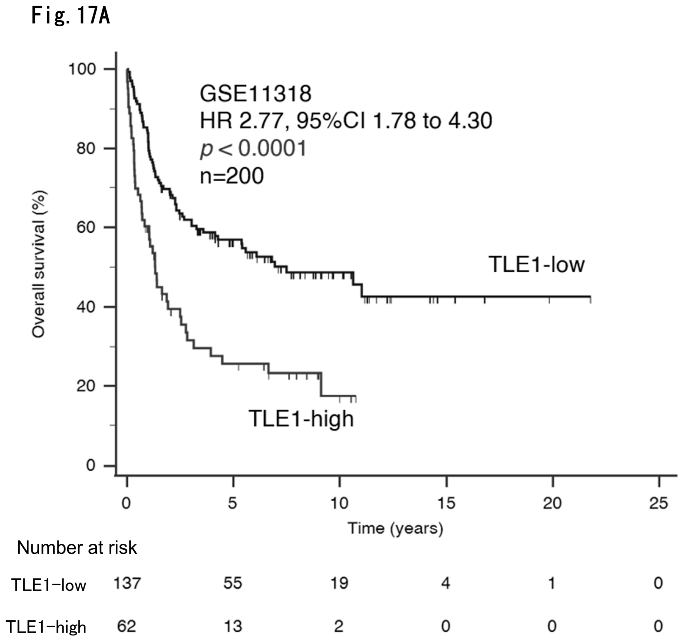

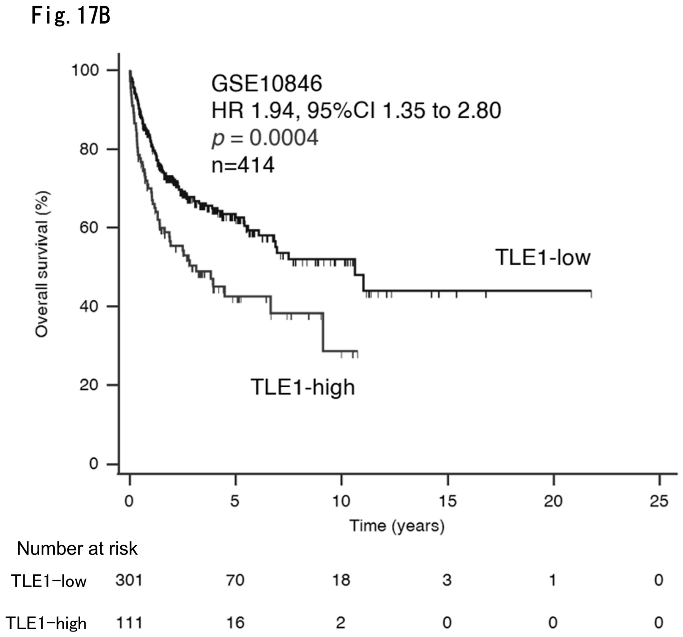

The results are shown in Figs. 17A-B. When divided into low and high TLE1 expression levels, it was found that the high expression level results in a poor prognosis. This event was similar in two different cohort analyses, suggesting that it is universal in B-cell lymphoma.

In the Examples above, it was revealed that BCV sensitivity in malignant tumors is associated with the expression level of TLE1. Specifically, it was shown that NK/T-cell lymphomas and T-cell lymphomas with a low expression level of TLE1 have high sensitivity to BCV. It was also shown that B-cell lymphomas with a high expression level of TLE1 have high sensitivity to BCV. Therefore, it is considered that by predicting sensitivity using the expression level of TLE1 as an indicator, a more appropriate BCV treatment can be implemented. For example, a patient's sensitivity to BCV can be examined using the expression level of TLE1 as an indicator, and BCV can be administered to a patient who is predicted to have high sensitivity. Hereby, an improvement in the therapeutic effect or an increase in the probability of a therapeutic effect appearing can be expected, as compared to when BCV is administered to an unspecified patient. Also, for example, when BCV is administered to a subject who is expected to have low TLE1 expression and high sensitivity to BCV, an improvement in the therapeutic effect or an increase in the probability of a therapeutic effect appearing can be expected, as compared to when BCV is administered to an unspecified patient. In addition, in patients with BCV-untreated NK/T lymphoma with low TLE1 expression or B-cell lymphoma with high TLE1 expression, the results showed a poor prognosis. Therefore, BCV treatment is considered to be an effective therapeutic method for malignant tumors with a poor prognosis. In addition, the antitumor effect of BCV was shown to be particularly high for T-cell lymphoma. Therefore, it was revealed that T-cell lymphoma is particularly suitable as a therapeutic target for BCV.

Hereinabove, the present invention has been described based on the Examples. It will be understood by those skilled in the art that the Examples are for illustrative purposes only and that various modifications are possible and such modifications are also within the scope of the present invention.

## Claims

1. A pharmaceutical composition for treatment of a malignant tumor, comprising brincidofovir, a pharmaceutically acceptable salt thereof, or a solvate thereof, wherein a subject of the treatment has a malignant tumor with low TLE1 expression.

2. The pharmaceutical composition according to claim 1, wherein the malignant tumor has low TLE1 expression and has high sensitivity to brincidofovir, a pharmaceutically acceptable salt thereof, or a solvate thereof.

3. The pharmaceutical composition according to claim 1 or 2, which suppresses proliferation of the malignant tumor cells.

4. The pharmaceutical composition according to any one of claims 1 to 3, wherein the malignant tumor is a hematologic cancer with low TLE1 expression.

5. The pharmaceutical composition according to any one of claims 1 to 4, wherein the malignant tumor is a lymphoma with low TLE1 expression.

6. The pharmaceutical composition according to any one of claims 1 to 5, wherein the malignant tumor is an NK/T-cell lymphoma with low TLE1 expression or a T-cell lymphoma with low TLE1 expression.

7. The pharmaceutical composition according to any one of claims 1 to 6, comprising brincidofovir.

8. A pharmaceutical composition for treatment of a malignant tumor, comprising brincidofovir, a pharmaceutically acceptable salt thereof, or a solvate thereof, wherein a subject of the treatment is a subject identified based on an expression level of TLE1 as an indicator.

9. The pharmaceutical composition according to claim 8, comprising brincidofovir.

10. The pharmaceutical composition according to claim 8 or 9, wherein the subject is a subject predicted to have high sensitivity to brincidofovir, a pharmaceutically acceptable salt thereof, or a solvate thereof, based on an expression level of TLE1 as an indicator.

11. The pharmaceutical composition according to any one of claims 8 to 10, wherein the subject is a subject identified based on an expression level of TLE1 in malignant tumor cells of the subject as an indicator.

12. The pharmaceutical composition according to any one of claims 8 to 11, wherein the treatment comprises testing an expression level of TLE1 of the subject.

13. The pharmaceutical composition according to any one of claims 8 to 12, wherein the treatment comprises identifying a subject having high sensitivity to brincidofovir, a pharmaceutically acceptable salt thereof, or a solvate thereof, based on an expression level of TLE1 as an indicator.

14. The pharmaceutical composition according to any one of claims 8 to 13, wherein the subject is a subject diagnosed as having a malignant tumor with low TLE1 expression.

15. The pharmaceutical composition according to any one of claims 8 to 13, wherein the subject is a subject diagnosed as having a malignant tumor with high TLE1 expression.

16. The pharmaceutical composition according to any one of claims 8 to 15, wherein the malignant tumor is a MYC-positive malignant tumor.

17. The pharmaceutical composition according to any one of claims 8 to 16, wherein the malignant tumor is a hematologic cancer.

18. The pharmaceutical composition according to any one of claims 8 to 17, wherein the malignant tumor is a lymphoma.

19. The pharmaceutical composition according to any one of claims 8 to 18, wherein the malignant tumor is an NK/T-cell lymphoma, a B-cell lymphoma, or a T-cell lymphoma.

20. The pharmaceutical composition according to claim 14, wherein the malignant tumor is an NK/T-cell lymphoma.

21. The pharmaceutical composition according to claim 14, wherein the malignant tumor is a T-cell lymphoma.

22. The pharmaceutical composition according to claim 15, wherein the malignant tumor is a B-cell lymphoma.

23. A composition for use in predicting sensitivity to brincidofovir, a pharmaceutically acceptable salt thereof, or a solvate thereof in a subject with a malignant tumor, the composition comprising a test reagent for an expression level of TLE1.

24. A composition for use in determining a subject with a malignant tumor to be administered brincidofovir, a pharmaceutically acceptable salt thereof, or a solvate thereof, the composition comprising a test reagent for an expression level of TLE1.

25. A composition for use in diagnosing whether a subject is a subject having a malignant tumor with high sensitivity to brincidofovir, a pharmaceutically acceptable salt thereof, or a solvate thereof, the composition comprising a test reagent for an expression level of TLE1.

26. A method for predicting sensitivity to brincidofovir, a pharmaceutically acceptable salt thereof, or a solvate thereof in a subject with a malignant tumor, the method comprising testing an expression level of TLE1 in a sample derived from the subject with the malignant tumor.

27. A method for determining a subject with a malignant tumor to be administered brincidofovir, a pharmaceutically acceptable salt thereof, or a solvate thereof, the method comprising testing an expression level of TLE1 in a sample derived from the subject with the malignant tumor.

28. A method for diagnosing whether a subject is a subject having a malignant tumor with high sensitivity to brincidofovir, a pharmaceutically acceptable salt thereof, or a solvate thereof, the method comprising testing an expression level of TLE1 in a sample derived from the subject with the malignant tumor.

29. A kit for use in the method according to any one of claims 26 to 28, the kit comprising a test reagent for an expression level of TLE1.

30. A composition for use in predicting a prognosis of an NK/T-cell lymphoma, the composition comprising a test reagent for an expression level of TLE1.

31. A method for predicting a prognosis of an NK/T-cell lymphoma, the method comprising predicting the prognosis based on an expression level of TLE1 in a sample from a subject with the NK/T-cell lymphoma.

32. The method according to claim 31, wherein low expression of TLE1 is predicted as a poor prognosis, or high expression of TLE1 is predicted as a good prognosis.

33. The method according to claim 31 or 32, further comprising predicting the prognosis of the NK/T-cell lymphoma based on an expression level of MYC in the sample.

34. A composition for use in selecting a method for treating an NK/T-cell lymphoma, the composition comprising a test reagent for an expression level of TLE1.

35. A method for selecting a method for treating an NK/T-cell lymphoma, the method comprising selecting the treatment method based on an expression level of TLE1 in a sample from a subject.

36. A composition for use in selecting a method for treating a B-cell lymphoma, wherein the treatment method comprises administering brincidofovir, a pharmaceutically acceptable salt thereof, or a solvate thereof to a subject, the composition comprising a test reagent for an expression level of TLE1.

37. A method for selecting a method for treating a B-cell lymphoma based on an expression level of TLE1 in a sample from a subject, wherein the treatment method comprises administering brincidofovir, a pharmaceutically acceptable salt thereof, or a solvate thereof to the subject.

38. A pharmaceutical composition for treatment of a malignant tumor, comprising brincidofovir, a pharmaceutically acceptable salt thereof, or a solvate thereof, wherein the malignant tumor is a T-cell lymphoma.

39. The pharmaceutical composition according to claim 38, wherein MYC expression in the malignant tumor is reduced by the treatment.

40. The pharmaceutical composition according to claim 38 or 39, wherein expression of INFβ1, INFγ, CCL5, CXCL10, or CD274 in the malignant tumor is increased by the treatment.

41. The pharmaceutical composition according to any one of claims 38 to 40, which suppresses proliferation of the T-cell lymphoma.

42. The pharmaceutical composition according to any one of claims 38 to 41, wherein the malignant tumor is a peripheral T-cell lymphoma.

43. The pharmaceutical composition according to any one of claims 38 to 42, comprising brincidofovir.
